(19) Europäisches Patentamt / European Patent Office / Office européen des brevets

(11) **EP 2 714 015 B1**

(12) **EUROPEAN PATENT SPECIFICATION**

(45) Date of publication and mention
of the grant of the patent:
**15.03.2017 Bulletin 2017/11**

(21) Application number: **12792447.0**

(22) Date of filing: **23.05.2012**

(51) Int Cl.:
**A61K 9/28** (2006.01)

(86) International application number:
**PCT/US2012/039152**

(87) International publication number:
**WO 2012/166474 (06.12.2012 Gazette 2012/49)**

(54) **CONTROLLED RELEASE SOLID DOSE FORMS**

FESTE DOSIERUNGSFORMEN MIT GESTEUERTER FREISETZUNG

FORMES POSOLOGIQUES SOLIDES À LIBÉRATION CONTRÔLÉE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **01.06.2011 US 201161519916 P**
**17.10.2011 US 201161547793 P**

(43) Date of publication of application:
**09.04.2014 Bulletin 2014/15**

(73) Proprietor: **FMC Corporation
Philadelphia, PA 19104 (US)**

(72) Inventors:
• **SIEPMANN, Juergen
59133 Phalempin (FR)**
• **CUPPOK, Yvonne
12205 Berlin (DE)**

(74) Representative: **Dunleavy, Kevin James et al
Mendelsohn Dunleavy, P.C.
p/o De Vries & Metman
Overschiestraat 180
1062 XK Amsterdam (NL)**

(56) References cited:
EP-B1- 1 095 651          WO-A1-2011/056934
US-A1- 2007 212 414       US-A1- 2007 264 346
US-A1- 2008 095 843       US-A1- 2010 092 556
US-A1- 2010 216 754       US-A1- 2010 310 667

• DATABASE MEDLINE [Online] US NATIONAL LIBRARY OF MEDICINE (NLM), BETHESDA, MD, US; June 2007 (2007-06), JI CHONG-MIN ET AL: "Guar gum/ethylcellulose coated pellets for colon-specific drug delivery.", XP002732032, Database accession no. NLM17702405 & JI CHONG-MIN ET AL: "Guar gum/ethylcellulose coated pellets for colon-specific drug delivery.", YAO XUE XUE BAO = ACTA PHARMACEUTICA SINICA JUN 2007, vol. 42, no. 6, June 2007 (2007-06), pages 656-662, ISSN: 0513-4870
• Cavalcanti: "Characterisation of ethylcellulose films containing natural polysaccharides by thermal analysis and FTIR spectroscopy", Acta Farm Bonaerense, 1 January 2004 (2004-01-01), XP55148776, Retrieved from the Internet: URL:http://www.latamjpharm.org/trabajos/23/1/LAJOP_23_1_1_8_0K5M9XR5RH.pdf [retrieved on 2014-10-24]

**Description**

**FIELD OF THE INVENTION**

The present invention is directed to controlled release solid dosage forms, controlled release film coating compositions, and methods for reducing the ethanol sensitivity of solid dosage forms.

**BACKGROUND OF THE INVENTION**

Controlled release dosage forms are designed to provide prolonged and/or delayed release of an active ingredient after the administration of the dosage form, as compared with the administration of an immediate release dosage form. Such sustained response offers many inherent therapeutic benefits that cannot be obtained with immediate release and short acting dose forms.

Controlled release dosage forms known in the art include beads, pellets, spheroids, coated capsules, coated tablets and ion exchange resins, wherein the sustained release of the active drug is realized via permeation of the active drug through a coating layer or a matrix formulation to slow down the release of the drug.

An essential characteristic of all controlled release dosage forms is the stability and consistency of the release profile, which must be documented in regulatory applications. The design of controlled release dosage forms must mitigate the risk of premature release ("dose dumping") leading to overdose. Coadministration of the dosage form with ethanol may accelerate release so reducing the sensitivity of the dosage form to the effect of ethanol is essential.

The sensitivity of controlled release dosage forms to ethanol is critical, for example, if the incorporated drug is highly potent, present at higher doses (than would be found in immediate release dose forms) and/or the undesired side effects are potentially severe. The co-ingestion of alcoholic beverages with solid dosage forms can lead to unintended high release rates and potentially fatal side effects. As a result, the sensitivity to ethanol has led to products being withdrawn from the market.

DATABASE MEDLINE, June 2007, Ji, Chong-Min et al., "Guar gum/ethycellulose coated pellets for colon-specific drug delivery," Database accession no. NLM17702405; XP002732032 and Ji Chong-Min et al., "Guar gum/ethylcellulose coated pellets for colon-specific drug delivery.", Yao Xue Xue Bao = ACTA Pharmaceutica Sinica, Vol. 42, no. 6, June 2007, p. 656-662 discloses coated pellets for colon-specific delivery. Varying ratios of ethylcellulose to guar gum were investigated to determine: the lag time of drug release and the release rate. It was determined that certain optimal ratios of ethylcellulose and guar gum enabled colon-specific release.

Cavalcanti: "Characterisation of ethylcellulose films containing natural polysaccharides by thermal analysis and FTIR spectroscopy," Acta Farm Bonaerense, 2004-01-01, XP55148776 characterises free films of ethylcellulose associated to inulin and guar gum or levan using thermal analysis and FTIR spectroscopy. Thermal analysis showed temperature dislocations of some parameters.

US 2007/212414 A1 discloses ethanol-resistant sustained release formulations that resist dose dumping in the presence of ethanol. The formulations include at least one heteropolysaccharide gum, at least one homopolysaccharide gum and at least one pharmaceutical diluent or includes at least one heteropolysaccharide gum, at least one cationic cross-linking compound selected from monovalent metal cations, multivalent metal cations and salts, and at least one pharmaceutical diluent.

The object of this invention was to identify a novel polymeric film coating, which has reduced sensitivity to high concentrations of ethanol in the surrounding bulk fluid.

The inventors have surprisingly found that the addition of small amounts of the specific guar gums of the present invention to ethylcellulose based film coatings effectively suppresses undesired acceleration of rapid drug release due to high ethanol concentrations. For example, theophylline release from matrix pellets coated with the aqueous ethylcellulose dispersion Aquacoat® ECD containing 10 and 15 % guar gum of the invention was unaffected in the presence of 40% ethanol in the release medium. Furthermore, the drug release of the coatings of the present invention have been found to be stable on long term and stressed storage.

**SUMMARY OF THE INVENTION**

The present invention is directed to a solid dose form comprising a film coating composition encapsulating a core, wherein: (i) the core comprises an active ingredient comprising at least one of a pharmaceutical, veterinary, or nutraceutical active ingredient; (ii) the film coating composition comprises ethylcellulose and guar gum, wherein the guar gum has an apparent viscosity $\geq$ 151.0 cps at a shear rate of 50 s$^{-1}$ in a 1% aqueous guar gum solution measured rotationally at 20°C after 1 minute equilibration using a 6 cm acrylic cone (1°) on a cone-plate viscometer wherein the shear is ramped up linearly from 1 to 50 s$^{-1}$ in 25 steps over 29 seconds; (iii) the dose form provides sustained release of the active ingredient; (iv) the guar gum is present in an amount greater than 5 wt% based on the weight of the guar gum and ethylcellulose; and (v) the dose form is ethanol resistant in that the release kinetics of said active ingredient are not significantly affected by the presence of alcohol, as determined by measuring a dissolution profile of said active ingredient in a USP 32 paddle apparatus operated with 900 ml at 37 °C and 100 rpm.

The present invention is also directed to sustained release film coating compositions for solid dose forms. The film coating compositions of the invention comprise ethylcellulose and guar gum, wherein the guar gum has an apparent viscosity $\geq$ 151.0 cps at a shear rate of 50 s$^{-1}$ in a 1% aqueous guar gum solution measured rotationally at 20°C after 1 minute equilibration using a 6 cm acrylic cone (1°) on a cone-plate viscometer wherein the shear is ramped up linearly from 1 to 50 s$^{-1}$ in 25 steps over 29 seconds. The film coating composition of the invention provides sustained release of and ethanol resistance to a pharmaceutical, veterinary or nutraceutical active ingredient contained within a solid dose form containing the film coating composition.

In another embodiment, the present invention is directed to a method of reducing the ethanol sensitivity of a pharmaceutical, nutraceutical or veterinary active ingredient in the core of a solid dosage form comprising coating the core with a film coating composition comprising ethylcellulose and guar gum, wherein the guar gum has an apparent viscosity $\geq$ 151.0 cps at a shear rate of 50 s$^{-1}$ in a 1% aqueous guar gum solution measured rotationally at 20°C after 1 minute equilibration using a 6 cm acrylic cone (1°) on a cone-plate viscometer wherein the shear is ramped up linearly from 1 to 50 s$^{-1}$ in 25 steps over 29 seconds. The film coating composition of the invention provides sustained release of and ethanol resistance to the active ingredient in the solid dose form.

## BRIEF DESCRIPTION OF THE DRAWINGS

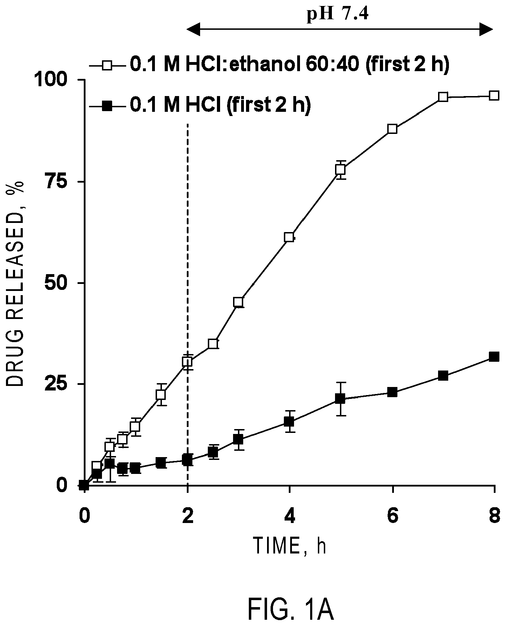

FIGS. 1A-1C show theophylline release from pellets coated (20 %) with the following: (a) ethylcellulose:*very low $\eta$* guar gum (having an apparent viscosity of 15 cps when measured using the stepped flow procedure described herein) 90:10, (b) ethylcellulose:*low $\eta$* guar gum (having an apparent viscosity of 52 cps when measured using the stepped flow procedure described herein) 90:10, or (c) ethylcellulose:*high $\eta$* guar gum (estimated to have an apparent viscosity of from 575-625 cps when measured using the stepped flow procedure described herein) 90:10 upon exposure to 0.1 M HCl for 2 hours and phosphate buffer pH 7.4 for the subsequent 6 h (filled squares), or 0.1 M HCl:ethanol 60:40 for 2 h and phosphate buffer pH 7.4 for the subsequent 6 hours (open squares).

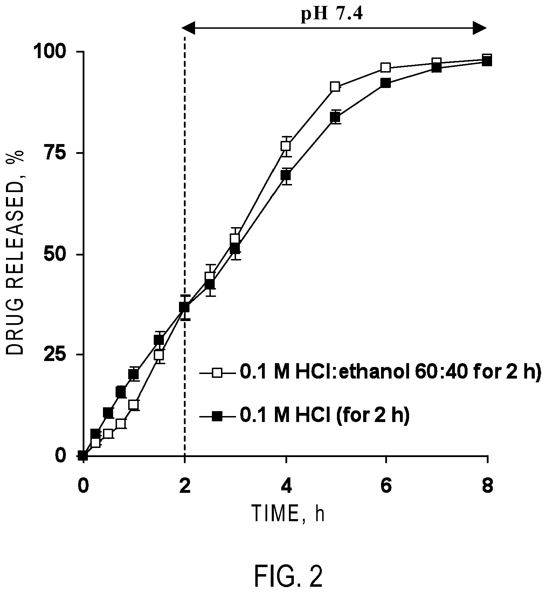

FIG. 2 shows theophylline release from drug matrix pellets coated with ethylcellulose:*medium $\eta$* guar gum (having an apparent viscosity of 320 cps when measured using the stepped flow procedure described herein) 85:15 (0.7 % guar gum in the total coating dispersion) upon exposure to: (i) 0.1 M HCl for 2 hours and phosphate buffer pH 7.4 for the subsequent 6 hours (filled squares), or (ii) 0.1 M HCl:ethanol 60:40 for 2 hours and phosphate buffer pH 7.4 for the subsequent 6 hours (open squares); (coating level: 20 %; 10 % talc).

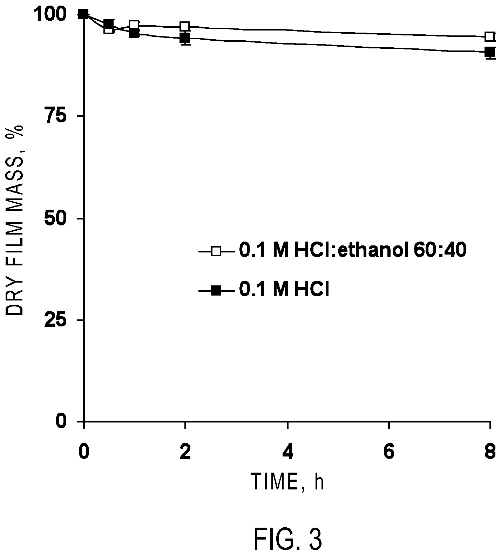

FIG. 3 shows dry mass loss of films containing ethylcellulose:*medium $\eta$* guar gum 85:15 upon exposure to 0.1 M HCl (filled squares) or 0.1 M HCl:ethanol 60:40 (open squares).

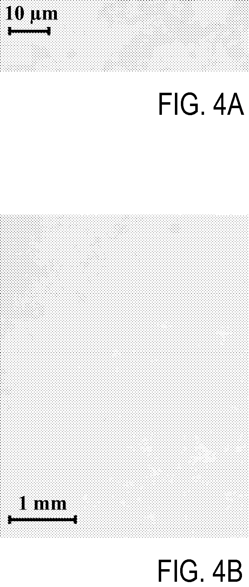

FIGS. 4A and 4B show compatibility of Aquacoat® ECD and guar gum. FIG. 4A shows a microscopic picture of an Aquacoat® ECD:*medium $\eta$* guar gum 85:15 aqueous dispersion stirred for 24 hours, and FIG. 4B shows a macroscopic picture of a thin film prepared from Aquacoat® ECD:*medium $\eta$* guar gum (polymer:polymer blend ratio = 85:15).

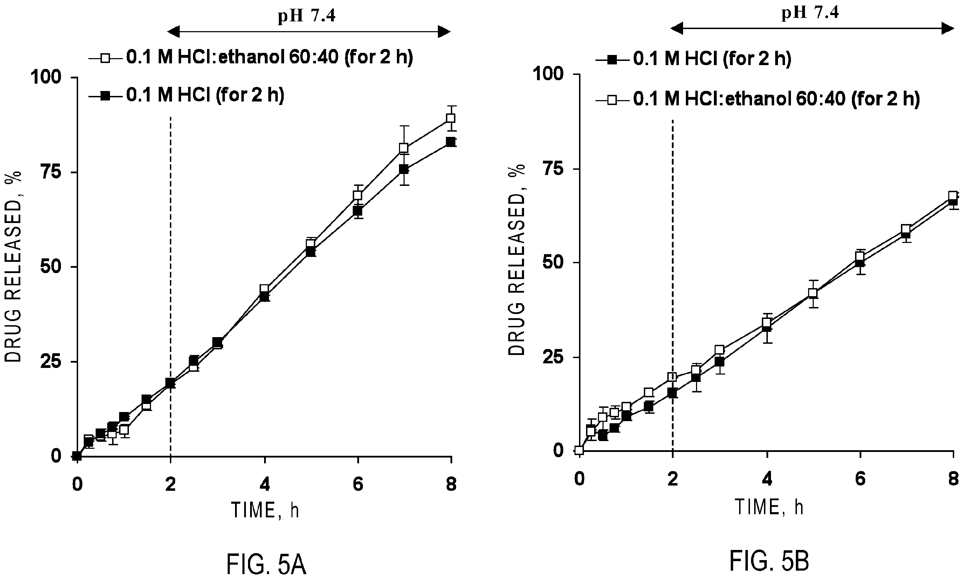

FIGS. 5A-5B show theophylline release from pellets coated with ethylcellulose:*medium $\eta$* guar gum 90:10 (1 % guar gum in the total coating dispersion) upon exposure to 0.1 M HCl for 2 hours and phosphate buffer pH 7.4 for the subsequent 6 hours (filled squares), or 0.1 M HCl:ethanol 60:40 for 2 hours and phosphate buffer pH 7.4 for the subsequent 6 hours (open squares) (coating level: 30 %). The aqueous dispersion contained: (a) 50 % talc (FIG. 5A), or (b) 20 % GMS (FIG. 5B).

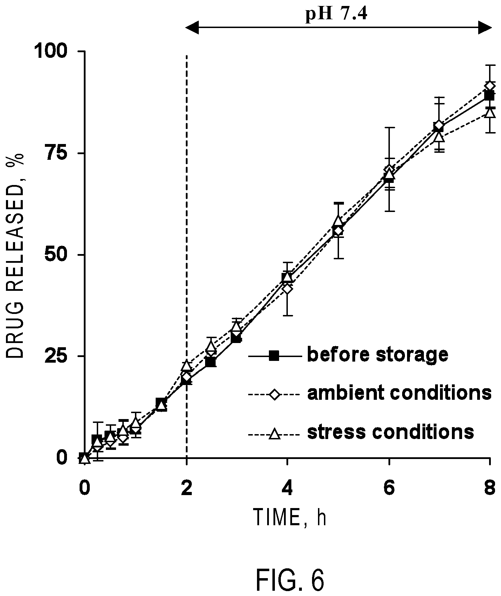

FIG. 6 shows storage stability of pellets coated with ethylcellulose:*medium $\eta$* guar gum 90:10 (1 % guar gum in the total coating dispersion). Theophylline release before and after 12 months storage at ambient or stress conditions was tested upon exposure to 0.1 M HCl:ethanol 60:40 for 2 hours and phosphate buffer pH 7.4 for the subsequent 6 hours (50 % talc; coating level: 30 %).

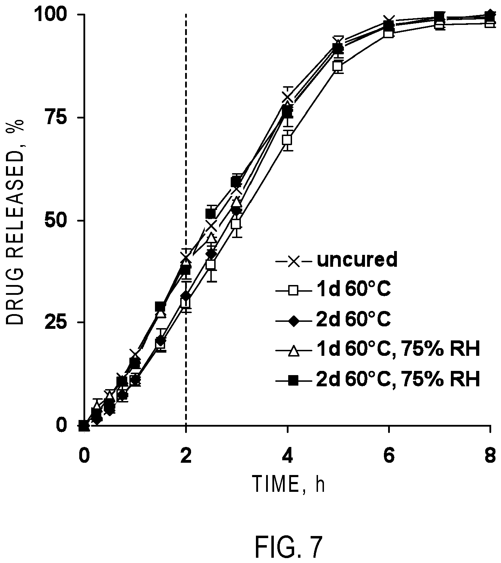

FIG. 7 shows the effects of the curing conditions (indicated in the Figure) on theophylline release from pellets coated with 20 % w/w ethylcellulose:*medium $\eta$* guar gum 85:15 (0.7 % guar gum in the total coating dispersion) in 0.1 M HCl:ethanol 60:40 for 2 h, followed by phosphate buffer pH 7.4 for the subsequent 6 h.

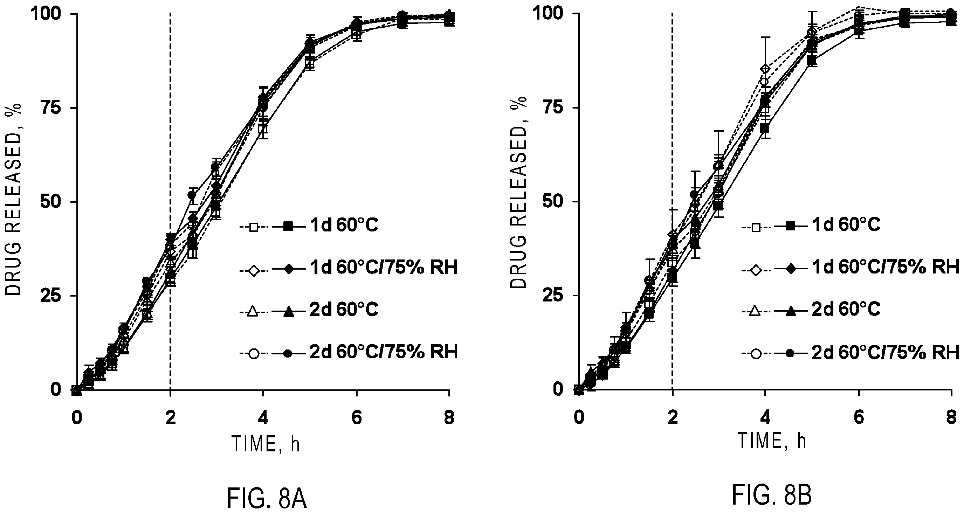

FIGS. 8A and 8B show the impact of the curing conditions (indicated in the figures: "1 d at 60 °C", "1 d at 60 °C & 75 % relative humidity", 2 d at 60 °C", or "2 d at 60 °C & 75 % relative humidity") on the storage stability of ethyl-cellulose:*medium* $\eta$ guar gum 85:15 (0.7 % guar gum in the total coating dispersion) coated pellets (coating level: 20 % w/w). More specifically, FIGS. 8A and 8B show theophylline release before storage (solid curves; closed symbols) and after 6 months open storage (dotted curves; open symbols) at: (a) ambient conditions (FIG. 8A), and (b) stress conditions (40 °C and 75% relative humidity; FIG. 8B). The release medium was 0.1 M HCl:ethanol 60:40 for the first 2 h, followed by phosphate buffer pH 7.4 for the subsequent 6 h.

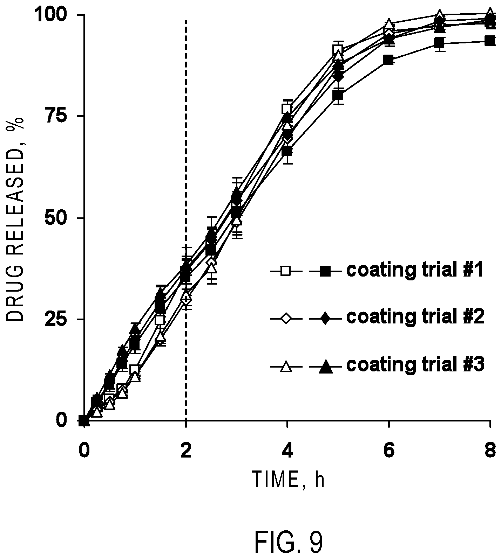

FIG. 9 shows the reproducibility of the coating process. Theophylline release is shown from three different pellet batches (coated with 20 % w/w ethylcellulose:*medium* $\eta$ guar gum 85:15, 0.7 % guar gum in the total coating dispersion) in 0.1 M HCl for 2 h, followed by phosphate buffer pH 7.4 (closed symbols) or 0.1 M HCl:ethanol 60:40 for 2 h, followed by phosphate buffer pH 7.4 (open symbols).

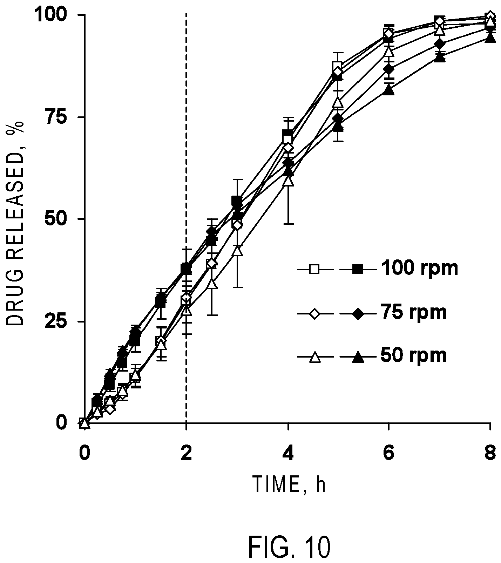

FIG. 10 shows the robustness of drug release with respect to the degree of agitation of the bulk fluid. Theophylline release is shown from pellets coated with 20 % w/w ethylcellulose:*medium* $\eta$ guar gum 85:15 (0.7 % guar gum in the total coating dispersion) in 0.1 M HCl for 2 h, followed by phosphate buffer pH 7.4 (closed symbols) or 0.1 M HCl:ethanol 60:40 for 2 h, followed by phosphate buffer pH 7.4 (open symbols) at different agitation speeds (as indicated).

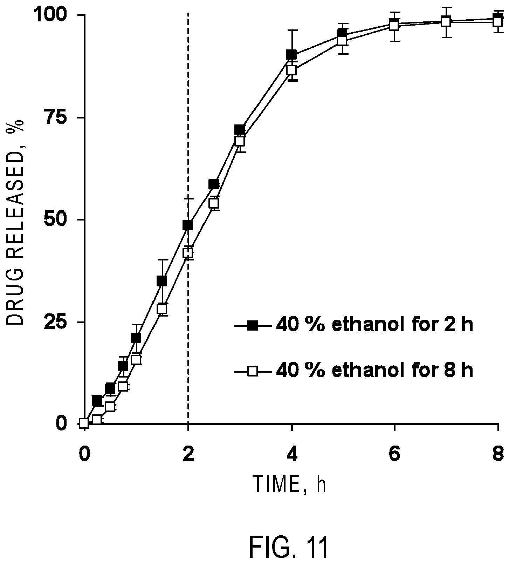

FIG. 11 shows the effects of the exposure time to ethanol. Theophylline release is shown from pellets coated with 20 % w/w ethylcellulose:*medium* $\eta$ guar gum 85:15 (0.7 % guar gum in the total coating dispersion) in 0.1 M HCl:ethanol 60:40 for 2 h, followed by phosphate buffer pH 7.4 (closed symbols) or in 0.1 M HCl:ethanol 60:40 for 8 h (open symbols).

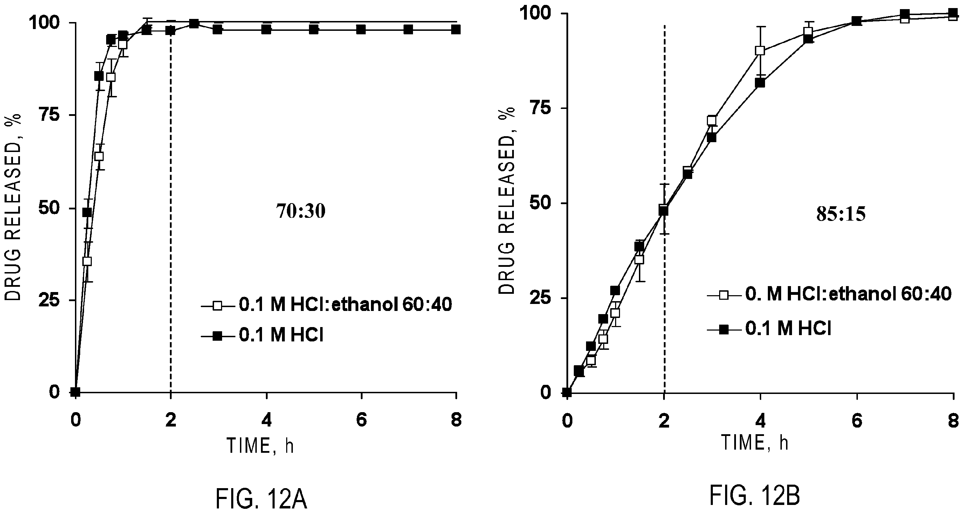

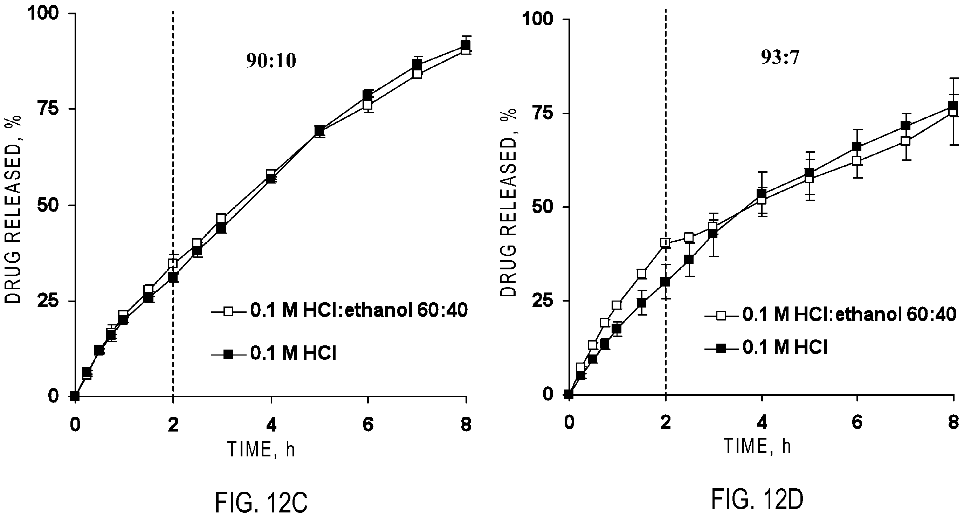

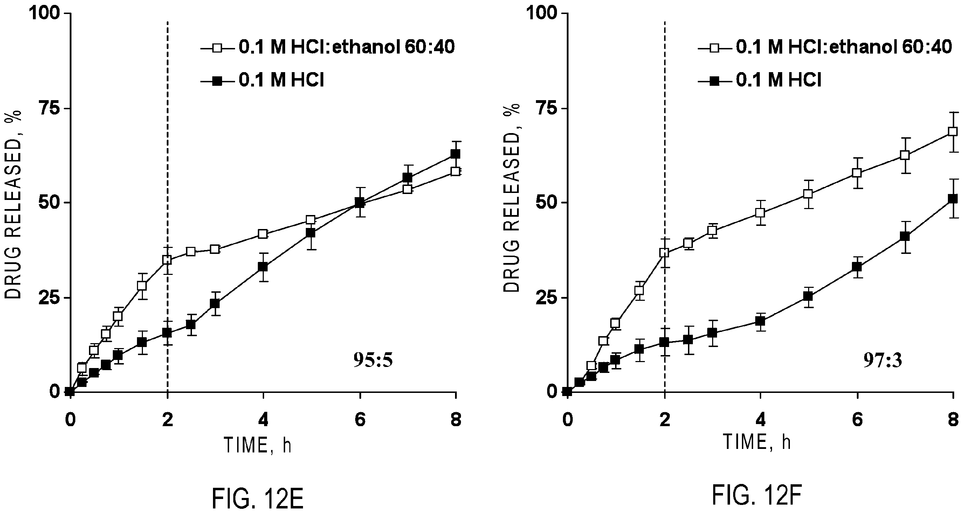

FIGS. 12A-12F show the impact of the ethylcellulose:*medium* $\eta$ guar gum blend ratio (indicated in the diagrams) (0.7 % guar gum in the total coating dispersion) on the sensitivity of drug release to ethanol. Theophylline release is shown from pellets (15 % coating level) in 0.1 M HCl for 2 h, followed by phosphate buffer pH 7.4 (closed symbols) or 0.1 M HCl:ethanol 60:40 for 2 h, followed by phosphate buffer pH 7.4 (open symbols).

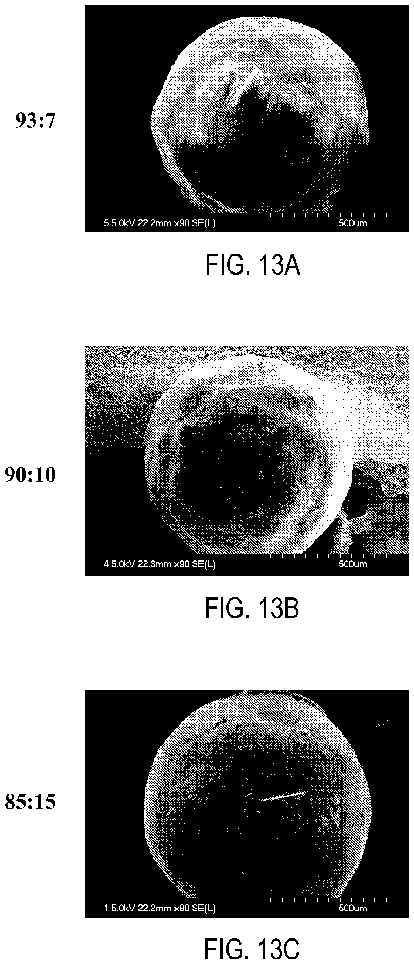

FIGS. 13A-13C show SEM pictures of pellets coated with different ethylcellulose:*medium* $\eta$ guar gum blend ratios (as indicated) (0.7 % guar gum in the total coating dispersion) (before exposure to the release medium).

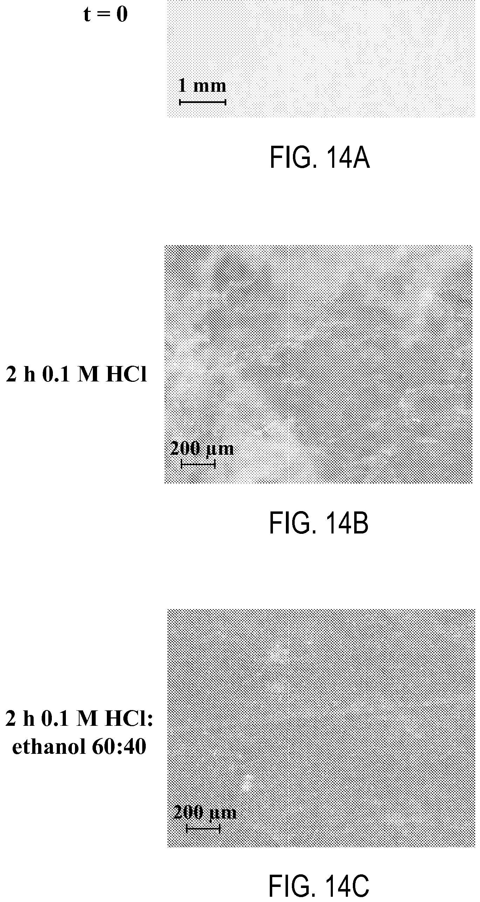

FIGS. 14A-14C show macroscopic pictures of free, polymeric films containing ethylcellulose:*medium* $\eta$ guar gum 85:15 at t=0 (FIG. 14A) and then after exposure to 0.1 M HCl (FIG. 14B) and 0.1 M HCl:ethanol 60:40 for 2 h (FIG. 14C) and subsequent drying (as indicated).

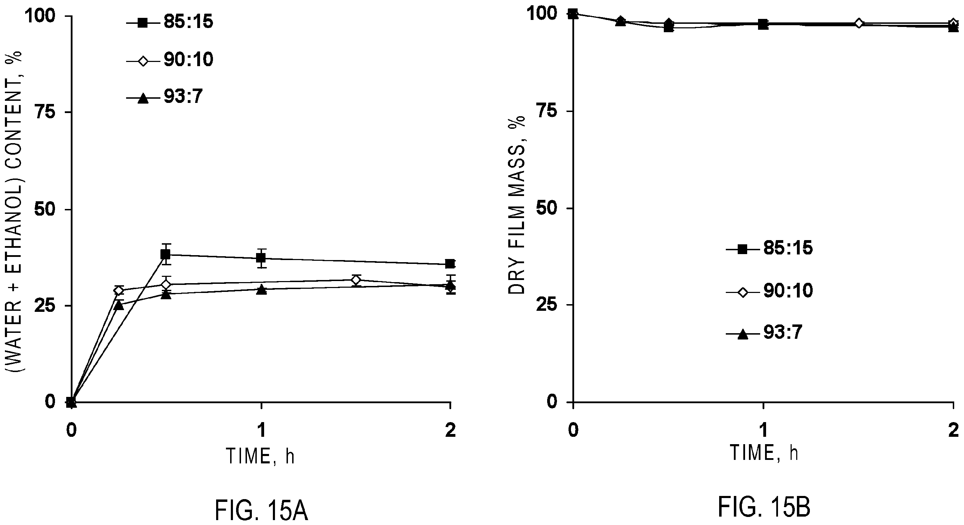

FIGS. 15A and 15B show the effects of the ethylcellulose:*medium* $\eta$ guar gum blend ratio (indicated in the diagrams) on changes in the: a) (water + ethanol) content (FIG. 15A), and b) dry mass of thin, free films upon exposure to 0.1 M HCl:ethanol 60:40 (FIG. 15B).

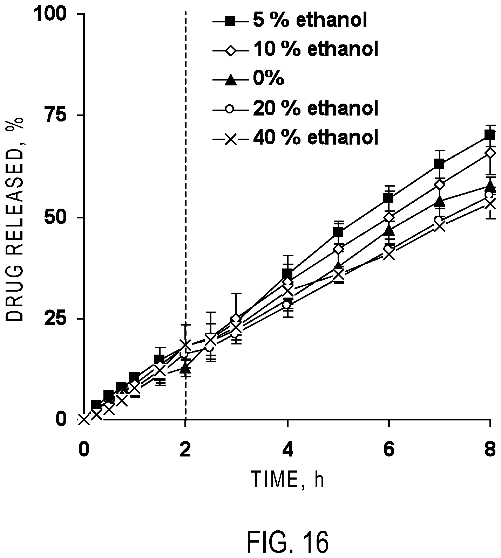

FIG. 16 shows the impact of the ethanol content in the bulk fluid (indicated in the diagram) on theophylline release from pellets coated with 20 % w/w ethylcellulose:*medium* $\eta$ guar gum 93:7 (1 % guar gum in total coating dispersion). The release medium was a 0.1 M HCl:ethanol mixture for the first 2 h, followed by phosphate buffer pH 7.4 for the subsequent 6 h.

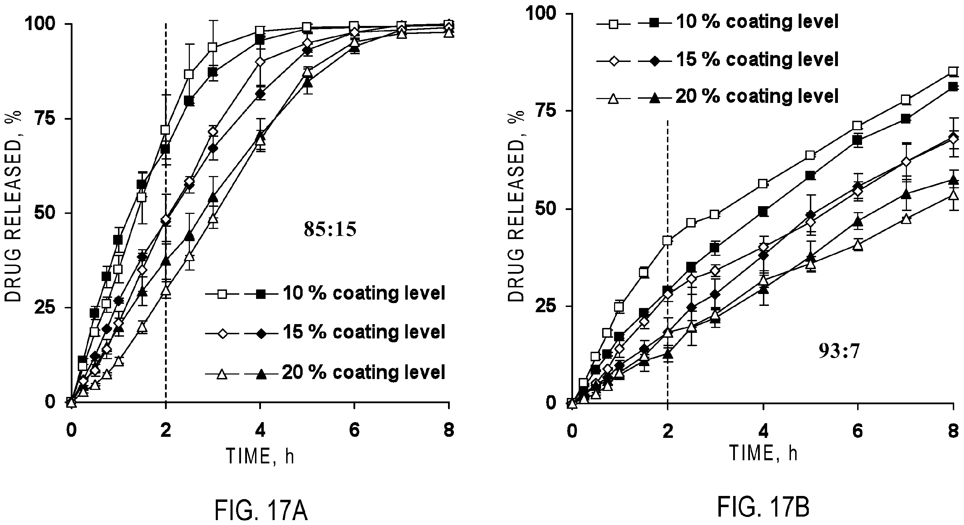

FIGS. 17A and 17B show the impact of the coating level (indicated in the diagrams) on theophylline release from pellets coated with:

a) ethylcellulose:*medium* $\eta$ guar gum 85:15 (0.7 % guar gum in total coating dispersion; FIG. 17A), or b) 93:7 (1 % guar gum in total coating dispersion; FIG. 17B) in (i) 0.1 M HCl for 2 h, followed by phosphate buffer pH 7.4 (closed symbols) or in (ii) 0.1 M HCl:ethanol 60:40 for 2 h, followed by phosphate buffer pH 7.4 (open symbols).

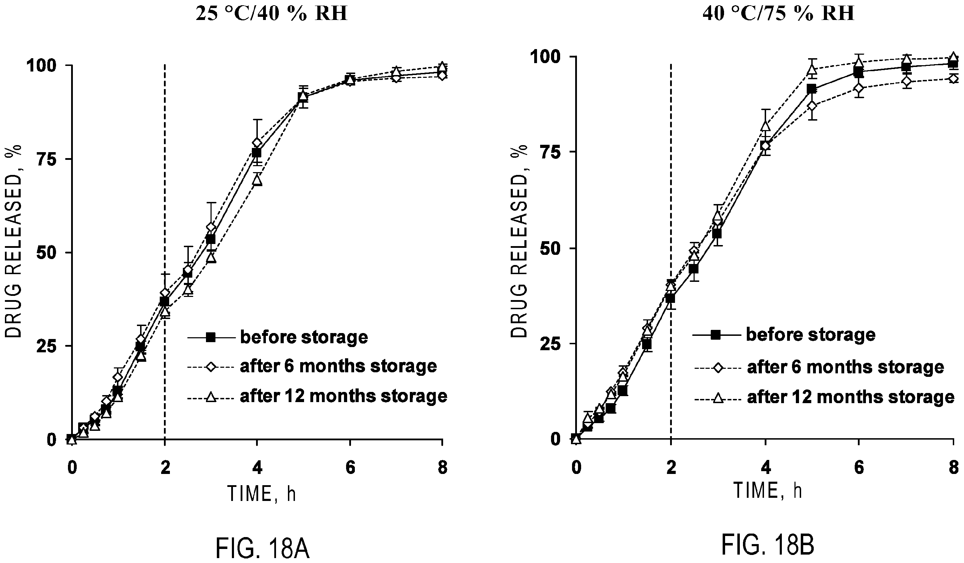

FIGS. 18A and 18B show the storage stability of theophylline pellets coated with 20 % w/w ethylcellulose:*medium* $\eta$ guar gum 85:15 (0.7 % guar gum in the total coating dispersion). Drug release is shown before (solid curves, closed symbols) and after 6 and 12 months open storage (dotted curves, open symbols) under: (a) ambient conditions (25 °C and 60 % relative humidity; FIG. 18A), or (b) stress conditions (40 °C and 75 % relative humidity; FIG. 18B) in 0.1 M HCl:ethanol 60:40 for 2 h, followed by phosphate buffer pH 7.4.

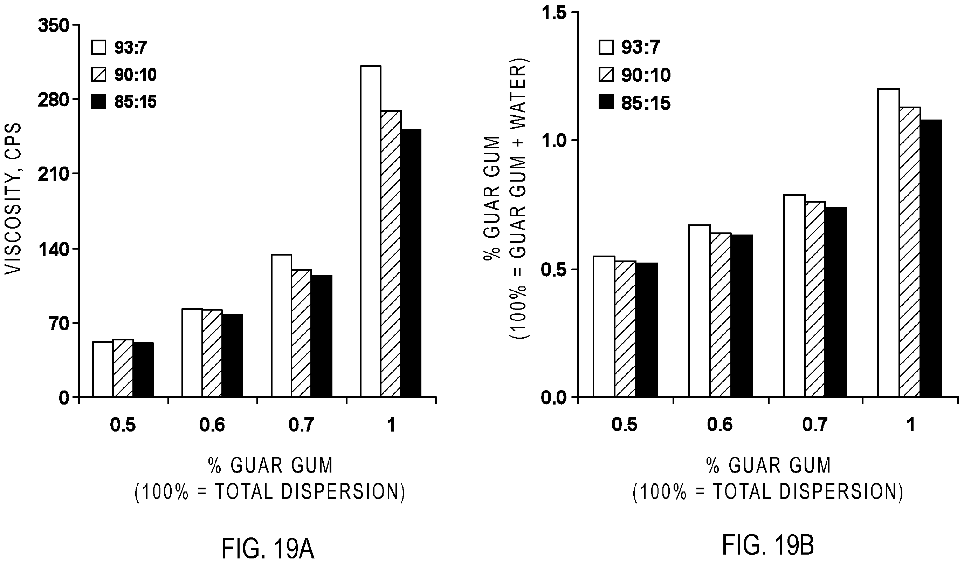

FIGS. 19A and 19B show the effects of the coating dispersion dilution on:

(a) the apparent viscosity (measured using the stepped flow procedure described herein) of the formulation sprayed onto the pellets (FIG. 19A), and (b) the percentage of *medium η* guar gum with respect to the "guar gum + total water" content of the formulation (FIG. 19B). The bars indicate the ethylcellulose:*medium η* guar gum blend ratio (93:7, 90:10 and 85:15 w/w, respectively).

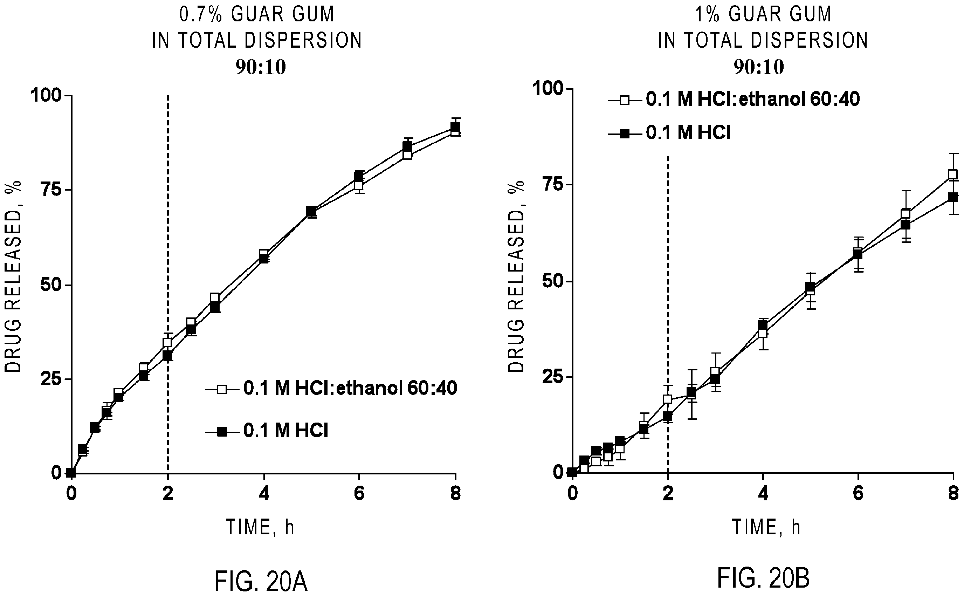

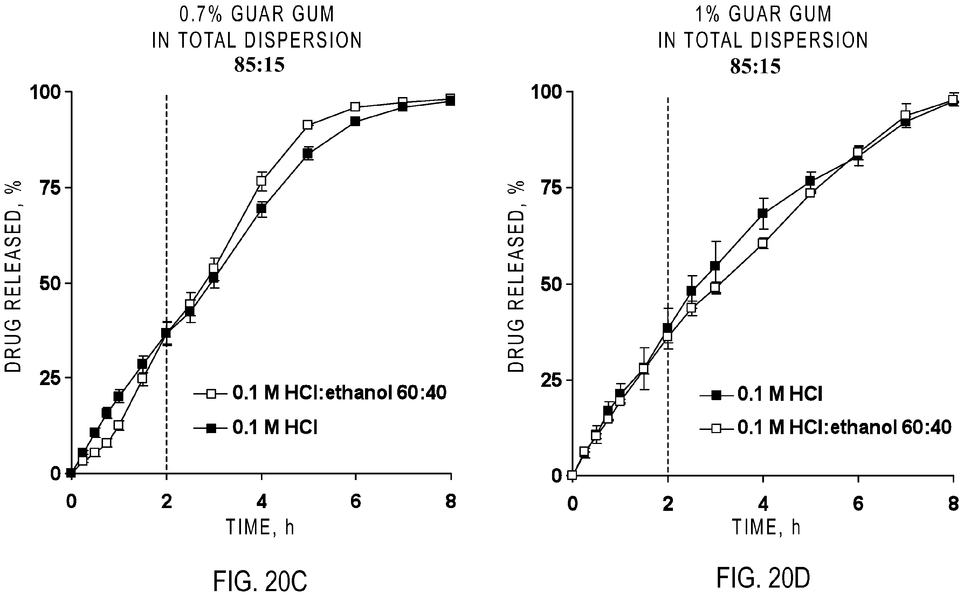

FIGS. 20A-20D show the impact of the guar gum dilution (0.7% versus 1%) on theophylline release from pellets coated with different ethylcellulose:*medium η* guar gum blends as indicated(coating level: 20 % w/w) in (i) 0.1 M HCl for 2 h, followed by phosphate buffer pH 7.4 (closed symbols) or in (ii) 0.1 M HCl:ethanol 60:40 for 2 h, followed by phosphate buffer pH 7.4 (open symbols).

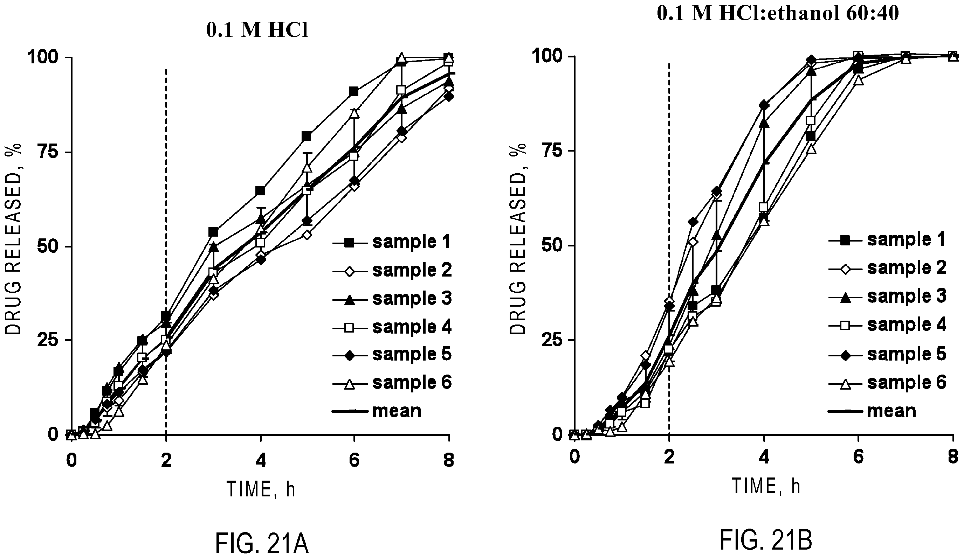

FIGS. 21A and 21B show theophylline release from *single* pellets coated with 20 % w/w ethylcellulose:*medium η* guar gum 85:15 (0.7 % guar gum in the total coating dispersion) in: a) 0.1 M HCl for 2 h, followed by phosphate buffer pH 7.4, or b) 0.1 M HCl:ethanol 60:40 for 2 h, followed by phosphate buffer pH 7.4. The thick curves show the respective mean values; error bars indicate standard deviations.

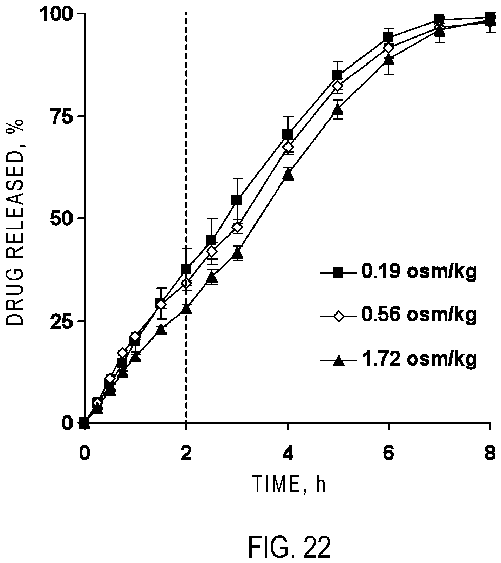

FIG. 22 shows the impact of the osmolality of the bulk fluid. Theophylline release is shown from pellets coated with 20 % w/w ethylcellulose:*medium η* guar gum 85:15 (0.7 % guar gum in the total coating dispersion) in 0.1 M HCl containing different amounts of NaCl for 2 h, followed by phosphate buffer pH 7.4.

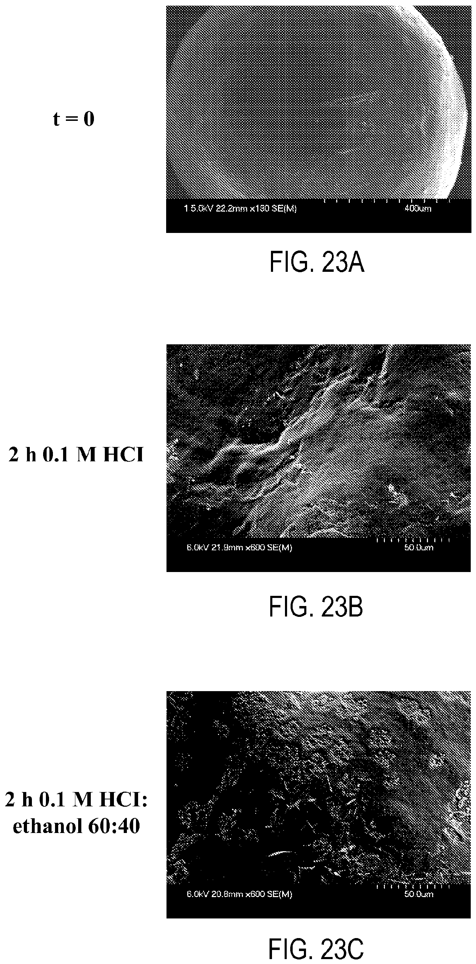

FIGS. 23A-23C show SEM pictures of surfaces of pellets coated with 20 % w/w ethylcellulose:*medium η* guar gum 85:15 (0.7 % guar gum in the total coating dispersion) at t=0 and after exposure to 0.1 M HCl or 0.1 M HCl:ethanol 60:40 for 2 h (and subsequent drying) (as indicated).

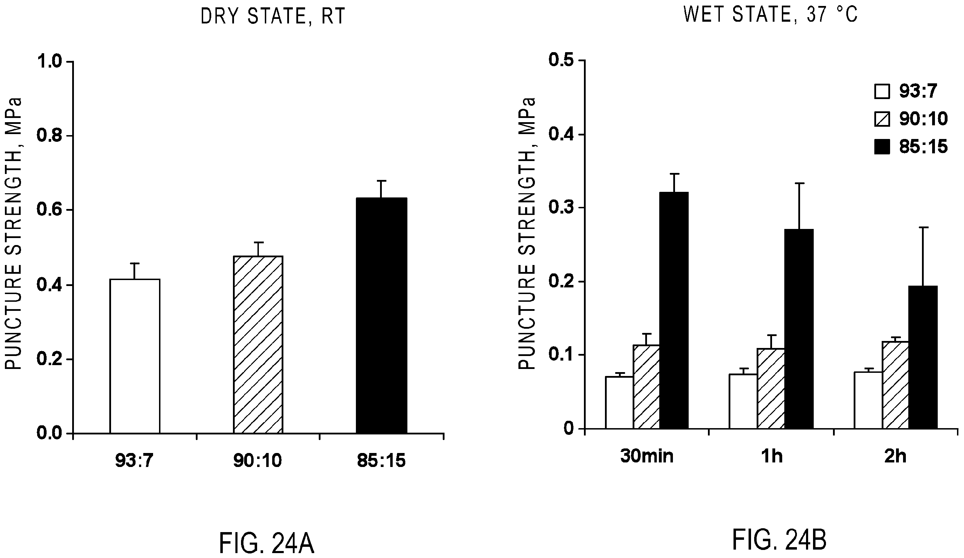

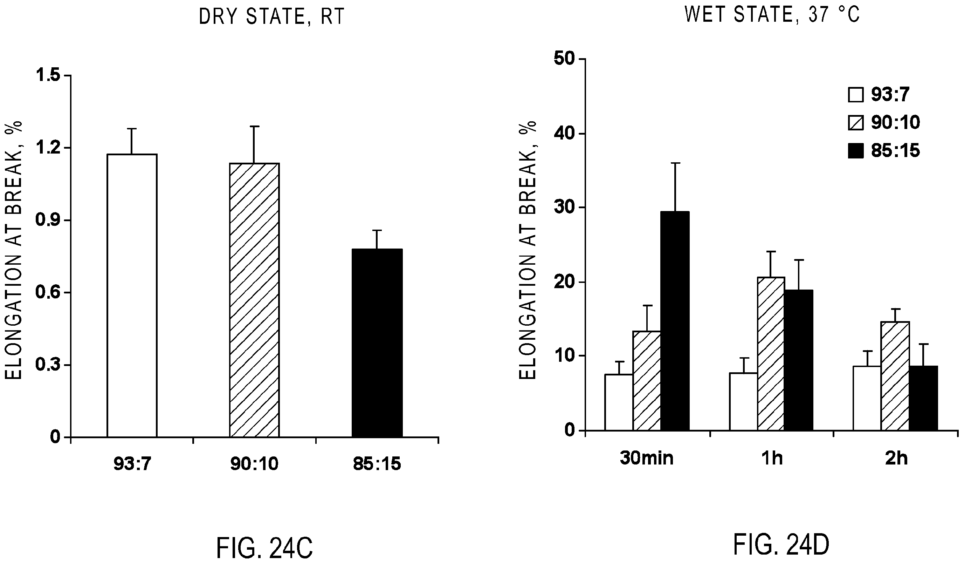

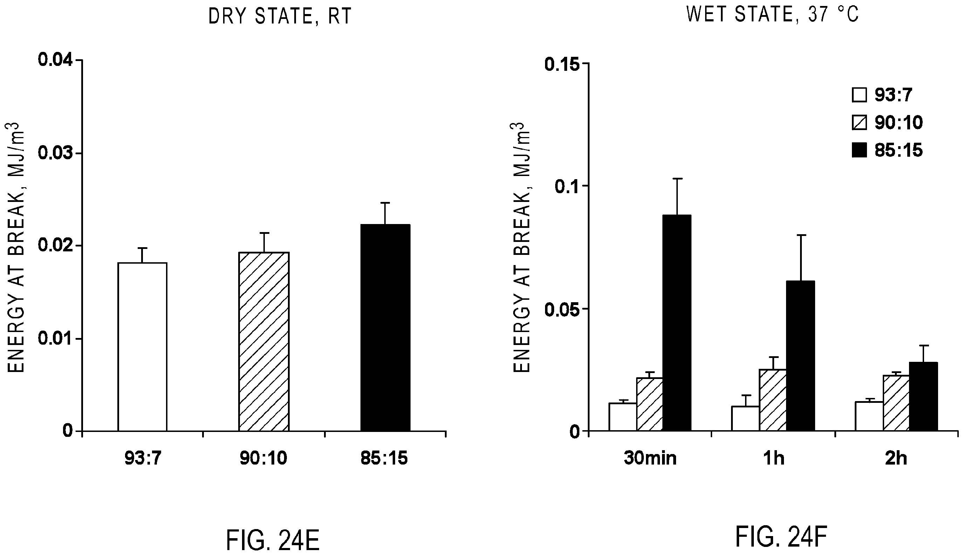

FIGS. 24A-24F show the mechanical properties of free, ethylcellulose:*medium η* guar gum films in the dry state at room temperature (FIGS. 24A, 24C and 24E) and in the wet state at 37 °C (FIGS. 24B, 24D and 24F) after exposure to 0.1 M HCl:ethanol 60:40 for different time periods (as indicated). The ethylcellulose:guar gum blend ratio is indicated in the diagrams.

## DETAILED DESCRIPTION OF THE INVENTION

"Dose dumping," as defined by the FDA, is the unintended, rapid release of a significant portion of a drug from a controlled release dosage form (Meyer, et al, "Awareness Topic: Mitigating the Risks of Ethanol Induced Dose Dumping From Oral Sustained/Controlled Release Dosage Forms," FDA's ACPS Meeting, October, 2005). This phenomenon can, for example, be caused by the consumption of alcoholic beverages, leading to high ethanol concentrations in the contents of the stomach (Roth et al., "Ethanol Effects on Drug Release From Verapamil Meltrex, an Innovative Melt Extruded Formulation," Int. J. Pharm., 368, 72-75, 2009). If drug release is controlled by a polymer, which is insoluble in water and the contents of the stomach under "normal" conditions, but soluble in aqueous media containing significant amounts of ethanol, the co-ingestion of alcoholic beverages can lead to unintended polymer dissolution. Thus, drug release can be rapid, instead of being controlled during prolonged periods of time. This is true for drug reservoirs, which are surrounded by release rate controlling polymeric films, as well as for drug matrix systems, in which the drug is embedded within a polymeric matrix. Surprisingly, the inventors have identified a coating composition containing ethylcellulose and a specific type of guar that reduces ethanol sensitivity in a solid dose form containing the film coating.

The solid dose forms of the present invention are ethanol resistant.This means that the release kinetics of the active ingredient are not significantly affected by the presence of alcohol. More specifically, as used herein, a solid dosage form is ethanol resistant (or not sensitive to ethanol) if the *in vitro* drug release data in 0.1 M HCl is compared with and without 40% ethanol for 2 hours at 37 °C and the difference throughout the two hour period in release profiles between the ethanol free media and ethanol containing media is (i) less than 15%, more preferably, less than 7.5%, when less than 20% of the active is released in the ethanol free media, and (ii) less than 30%, more preferably, less than 15%, when between 20 and 40%, preferably, 20-50%, more preferably, 20-80%, of the active is released in ethanol free media. A typical apparatus for determining the dissolution profile is USP 32 paddle apparatus (900 ml, 37 °C, 100 rpm).

In addition to being ethanol resistant throughout the two hour period as noted in the foregoing definition, the present invention has also been found to be ethanol resistant meeting the foregoing release profile definition when subsequently and immediately placed (after the two hour period in 0.1 M HCl with and without 40% ethanol at 37 °C) in phosphate buffer at pH 7.4 at 37 °C for at least three hours, at least four hours, at least five hours, at least six hours, at least seven hours, and at least eight hours; i.e., the difference in release profiles between the ethanol free media and ethanol containing media throughout the two hour period in 0.1 M HCl (with and without 40% ethanol) at 37 °C and thereafter throughout the at least three hour period (or, the at least four hour period, five hour period, six hour period, seven hour period or eight hour period) in phosphate buffer at pH 7.4 at 37 °C is (i) less than 15%, more preferably, less than 7.5%, when less than 20% of the active is released in the ethanol free media, and (ii) less than 30%, more preferably, less than 15%, when between 20 and 40%, preferably, 20-50%, more preferably, 20-80%, of the active is released in ethanol free media.

Immediate release of drug is often considered to be greater than 85% of the drug released in less than 15 minutes when measured *in vitro* in accordance with the following standard test: the dosage form is exposed to 900 mL 0.1 M HCl in a USP 32 paddle apparatus (37 °C, 100 rpm). At pre-determined time points, samples are withdrawn and their drug contents analyzed using an appropriate analytical technique for the respective drug. Controlled release, as used herein, encompasses any release profile that is not immediate release and includes less than 85% drug released in greater than 15 minutes and 100% drug released in, for example, 2 hours, 4 hours, 6 hours or anywhere from 8 to 12 hours or longer all as measured with the following test: the dosage form is exposed to 900 mL 0.1 M HCl in a USP 32 paddle apparatus (37 °C, 100 rpm). At pre-determined time points, samples are withdrawn and their drug contents analyzed using an appropriate analytical technique for the respective drug. Optionally, the release medium is partially or completely replaced after more than 1 h, e.g., completely replaced by phosphate buffer pH 7.4 (USP 32, 37 °C, 100 rpm) after 2 hours. Controlled release, as used herein, includes delayed release, enteric release, pulsatile release, sustained release, programmed release rates, and extended release.

In one embodiment, the present invention is directed to a solid dose form comprising a film coating composition encapsulating a core, wherein: (i) the core comprises an active ingredient comprising at least one of a pharmaceutical, veterinary, or nutraceutical active ingredient; (ii) the film coating composition comprises ethylcellulose and guar gum, wherein the guar gum has an apparent viscosity ≥ 151.0 cps at a shear rate of 50 s$^{-1}$ in a 1% aqueous guar gum solution measured rotationally at 20°C after 1 minute equilibration using a 6 cm acrylic cone (1°) on a cone-plate viscometer wherein the shear is ramped up linearly from 1 to 50 s$^{-1}$ in 25 steps over 29 seconds; (iii) the dose form provides sustained release of the active ingredient; (iv) the guar gum is present in an amount greater than 5 wt% based on the weight of the guar gum and ethylcellulose; and (v) the dose form is ethanol resistant.

In the present invention, the ethylcellulose is used in an aqueous dispersion. Typical aqueous dispersions can contain 20-40 wt% ethylcellulose. Commercially available ethylcellulose aqueous dispersions are, for example, available from FMC Corporation and sold under the name Aquacoat® ECD and from Colorcon sold under the name Surelease®. Aquacoat® ECD is an aqueous dispersion containing 30% by weight ethylcellulose.

Guar gum is a natural polysaccharide extracted from the seeds of *cyamopsis tetragonolobus.* Importantly, guar gum is soluble in water. Consequently, pure guar gum coatings do not allow for controlled oral drug delivery. The guar gum is typically dissolved in water and then added to the aqueous dispersion containing the ethylcellulose.

The guar gum used in the present invention has an apparent viscosity ≥ 151.0 cps at a shear rate of 50 s$^{-1}$ in a 1% aqueous guar gum solution measured rotationally at 20°C after 1 minute equilibration using a 6 cm acrylic cone (1°) on a cone-plate viscometer, wherein the shear is ramped up linearly from 1 to 50 s$^{-1}$ in 25 steps over 29 seconds. Such guar gums are typically considered to have medium to high molecular weights.

The apparent viscosity of the guar gum of the invention is measured at a shear rate of 50 s$^{-1}$ in a 1% aqueous guar gum solution measured rotationally at 20°C after 1 minute equilibration using a 6 cm acrylic cone (1°) on a cone-plate viscometer, wherein the shear is ramped up linearly from 1 to 50 s$^{-1}$ in 25 steps over 29 seconds. A typical example of such a cone-plate viscometer is an AR2000Ex rheometer (TA Instruments, New Castle, USA). This procedure is often referred to as a stepped flow procedure. Unless otherwise indicated, all viscosities referred to herein are apparent viscosities obtained using this specific stepped flow procedure.

Typical guar gums useful in the present invention have an apparent viscosity of from 151.0 to 2,000 cps, more particularly, from 151.0 to 1,250 cps, more particularly, from 151.0 to 1,000 cps, more particularly, from 250 to 1,250 cps, and, more particularly, from 250 to 1,000 cps, and mixtures thereof, when measured using the stepped flow procedure described herein. Further examples of the guar gum that can be used in the present invention have an apparent viscosity of from 320 to 2,000 cps, more particularly, from 320 to 1,250 cps, and, more particularly, from 320 to 1,000 cps, and mixtures thereof, when measured using the stepped flow procedure described herein. It is possible that the guar gum component of the present invention contains a specific guar gum having an apparent viscosity below 151.0 cps (as measured herein), provided such a guar gum is blended with other guar gums to obtain an apparent viscosity (of all the combined guar gums) ≥ 151.0 cps (as measured herein).

The guar gum of the invention is used in an amount of at least 5% based on the weight of the guar gum and ethylcellulose. The weight % ratio of the ethylcellulose to guar gum typically used in the present invention may be any one of the following including any and all ranges by and between the following: 60:40; 61:39; 62:38; 63:37; 64:36; 65:35; 66:34; 67:33; 68:32; 69:31; 70:30; 71:29; 72:28; 73:27; 74:26; 75:25; 76:24; 77:23; 78:22; 79:21; 80:20; 81:19; 82:18; 83:17; 84:16; 85:15; 86:14; 87:13; 88:12; 89:11; 90:10; 91:9; 92:8; 93:7; and 94:6; all weight % ratios are ethylcellulose to guar, respectively. For example, the weight % ratio of the ethylcellulose to guar gum typically used in the present invention is from 60:40 to less than 95:5, respectively; more specifically, 60:40 to 93:7, respectively; 70:30 to 93:7, respectively; 75:25 to 93:7, respectively; 75:25 to 92:8, respectively; 75:25 to 90:10, respectively; 80:20 to 93.7, respectively; 80:20 to 92:8, respectively; 80:20 to 90:10, respectively; 85:15 to 92:8, respectively; and 85:15 to 90:10, respectively.

The film coating composition of the invention may contain a plasticizer. The plasticizer may reduce the glass transition temperature (Tg) so that films formed at a suitable film forming temperature are softer, more ductile, and have increased mechanical stress. The plasticizer may also act as a good swelling agent for the coating dispersion. Examples of suitable plasticizers include dibutyl sebacate, diethyl phthalate, acetyltriethyl citrate, triethyl citrate, tibutyl citrate, triacetin, acetylated monoglycerides, phthalate esters, castor oil, etc. Triethyl citrate and dibutyl sebacate are especially preferred plasticizers for use in the aqueous dispersions of this invention. When used, the plasticizer is typically added to the ethylcellulose aqueous dispersion after the ethylcellulose dispersion is prepared using known techniques and is present in a typical amount of about 1 to about 50 % by weight of the ethylcellulose.

The film coating composition may also contain a stabilizer that decreases the surface energy of the aqueous ethylcellulose dispersion. Examples include surfactants such as sodium dodecyl sulfate and cetyl alcohol.

The film coating composition may also contain an anti-tacking agent, such as talc, to reduce sticking during coating.

Typically, the active ingredient is present in the solid dosage form in an amount of from 1 $\mu$g to 1 g.

The coating of the present invention may be coated on a wide variety of cores, such as pellets, tablets, soft capsules, hard capsules, powders, granules, beads, films and film-enrobed dosage forms, microspheres, seeds, ion-exchange resin beads, and other single unit or multi-particulate systems, in order to obtain a desired controlled release of the therapeutically active agent. Granules, spheroids, or pellets, etc., prepared in accordance with the present invention can be presented in a capsule or film-enrobed dosage form or in any other suitable dosage form. They can be mixed with other drug preparations, or they can be mixed with other vehicles and drugs or particles that contain drugs or particles that have been subjected to film coating, after which they can be compressed into tablets or filled into capsules.

A wide variety of therapeutically active agents can be used in conjunction with the present invention. The therapeutically active agents (e.g. pharmaceutical agents) which may be used in the compositions of the present invention include both water soluble and water insoluble drugs. Examples of such therapeutically active agents include antihistamines (e.g., dimenhydrinate, diphenhydramine, chlorpheniramine and dexchlorpheniramine maleate), analgesics (e.g., aspirin, codeine, morphine, dihydromorphone, oxycodone, etc.), anti-inflammatory agents (e.g., naproxyn, diclofenac, indomethacin, ibuprofen, acetaminophen, aspirin, sulindac), gastro-intestinals and anti-emetics (e.g., metoclopramide), anti-epileptics (e.g., phenytoin, meprobamate and nitrezepam), vasodilators (e.g., nifedipine, papaverine, diltiazem and nicardirine), anti-tussive agents and expectorants (e.g., codeine phosphate), anti-asthmatics (e.g. theophylline), anti-spasmodics (e.g. atropine, scopolamine), hormones (e.g., insulin, leparin), diuretics (e.g., eltacrymic acid, bendrofluazide), anti-hypotensives (e.g., propranolol, clonidine), bronchodilators (e.g., albuterol), anti-inflammatory steroids (e.g., hydrocortisone, triamcinolone, prednisone), antibiotics (e.g., tetracycline), antihemorrhoidals, hypnotics, psychotropics, antidiarrheals, mucolytics, sedatives, decongestants, laxatives, antacids, vitamins, stimulants (including appetite suppressants such as phenylpropanolamine) and mixtures thereof. The above list is not meant to be exclusive.

In certain preferred embodiments, the therapeutically active agent comprises hydromorphone, oxycodone, dihydrocodeine, codeine, dihydromorphine, morphine, buprenorphine, salts of any of the foregoing, and mixtures of any of the foregoing, and the like. In one preferred embodiment, the therapeutically active agent comprises aspirin, ibuprofen, or acetaminophen and their mixtures with other pharmaceutically compatible, therapeutically active agents.

When the sustained release coating of the present invention is to be applied to tablets, the tablet core (e.g. the substrate) may comprise the active agent along with any pharmaceutically accepted inert pharmaceutical filler (diluent) material, including, but not limited to, sucrose, dextrose, lactose, microcrystalline cellulose, xylitol, fructose, sorbitol, mixtures thereof and the like. Also, an effective amount of any generally accepted pharmaceutical lubricant, including calcium or magnesium salts may be added to the above-mentioned ingredients of the excipient prior to compression of the tablet core ingredients. Most preferred is magnesium stearate in an amount of about 0.5-3% by weight of the solid dosage form.

In another embodiment, the present invention is also directed to the sustained release film coating compositions described herein.

In a further embodiment, the present invention is directed to a method of reducing the ethanol sensitivity of a pharmaceutical, nutraceutical or veterinary active ingredient in the core of a solid dosage form comprising coating the core with the film coating composition described herein. The film coating composition provides sustained release of and ethanol resistance to the active ingredient.

The process for making, using and coating the film coating composition on the solid dosage form can be any of those known in the field. An example of film coating preparations and coating processes are disclosed in US 7,829,148 (incorporated herein by reference). The dispersion containing the ethylcellulose and guar gum is typically coated on a dry solids basis in an amount of 2 to 40%, preferably 10-20%, more preferably, 10-15%, by weight of the total dose form.

The present invention is now described in more detail by reference to the following examples, but it should be understood that the invention is not construed as being limited thereto. Unless otherwise indicated herein, all parts, percents, ratios and the like are by weight.

EXAMPLES

**EXAMPLE 1**

*Materials*

Theophylline matrix pellets (70 % drug content, diameter: 0.71-1.25 mm; FMC BioPolymer, Philadelphia, PA, USA); Ethylcellulose Aqueous Dispersion NF (Aquacoat® ECD 30D; FMC BioPolymer); *very low viscosity* guar gum (i.e., *very low $\eta$* guar gum, apparent viscosity of a 1 % aqueous guar gum = 15 cps; TIC Pretested Nutriloid215 LV powder; TIC Gums, Belcamp, MD, USA); *low viscosity* guar gum (i.e., *low $\eta$* guar gum, apparent viscosity of a 1 % aqueous guar gum solution = 52 cPs; TIC Pretested Gum Guar TICOLV FCC Powder; TIC Gums); *medium viscosity* guar gum (i.e., *medium $\eta$* guar gum, apparent viscosity of a 1 % aqueous guar gum solution = 320 cPs; Polygum 240/80; Polygal Trading, Maerstetten, Switzerland); *high viscosity* guar gum (i.e., *high $\eta$* guar gum, estimated to have an apparent viscosity of from about 575-625 cPs when tested using the stepped flow procedure described herein; Guar HV 225; Alland & Robert, Port-Mort France, France); dibutyl sebacate (DBS; Morflex, Greensboro, NC, USA); ethanol (Fisher Bioblock Scientific, Illkirch, France); glyceryl monostearate ("GMS"; Cutina GMS V PH; Cognis, Duesseldorf, Germany); talc (Luzenac Val Chisone, Porte, Italy); polysorbate 80 (Montanox 80; Seppic, Paris, France). All apparent a 1% aqueous guar gum solution measured rotationally at 20°C after 1 minute equilibration using a 6 cm acrylic cone (1°), wherein the shear was ramped up linearly from 1 to 50 s$^{-1}$ in 25 steps over 29s. All coating levels, unless otherwise indicated, refer to the amount of the coating (wt%) on a dry solids basis by weight of the uncoated dose form.

*Preparation and characterization of thin polymeric films*

Aquacoat® ECD was plasticized for 1 day with 25 % DBS (w/w; based on the ethylcellulose mass). Guar gum was dissolved in purified water (*medium $\eta$*: 0.7 % w/w, 100 % reference value = total formulation to be cast; 2 h stirring). The two liquids were blended and stirred for 30 minutes prior to use. Films were prepared by casting Aquacoat® ECD:guar gum blends onto Teflon plates and subsequent controlled drying for 24 hours at 60 °C.

The water uptake and dry mass loss kinetics of the films were determined as follows: pieces of 5 cm x 5 cm were placed into 100 mL plastic containers filled with 100 mL pre-heated release medium (0.1 M HCl or 0.1 M HCl:ethanol 60:40), followed by horizontal shaking (37 °C, 80 rpm; GFL 3033, Gesellschaft fuer Labortechnik, Burgwedel, Germany). At predetermined time points, samples were withdrawn, accurately weighed [wet mass(t)] and dried to constant mass at 60 °C [dry mass (t)]. The water content (%) and dry film mass (%) at time t were calculated as follows:

$$\text{water content } (\%) \, (t) = \frac{\text{wet mass (t)} - \text{dry mass (t)}}{\text{wet mass (t)}} \cdot 100 \, \% \tag{1}$$

$$\text{dry film mass } (\%) \, (t) = \frac{\text{dry mass (t)}}{\text{dry mass (0)}} \cdot 100 \, \% \tag{2}$$

The mechanical properties of the films (puncture strength, percent elongation and energy at break) in the dry and wet state were measured using the puncture test and a texture analyzer (TAXT.Plus, Swantech, Villeneuve la Garenne, France). Film specimens were mounted on a film holder (n = 6). The puncture probe (spherical end: 5 mm diameter) was fixed on the load cell (5 kg) and driven downward with a cross-head speed of 0.1 mm/s to the center of the film holder's hole (diameter: 10 mm). Load versus displacement curves were recorded until rupture of the film and used to determine the mechanical properties as follows:

$$puncture\ strength = \frac{F}{A} \qquad\qquad (3)$$

where F is the load required to puncture the film; A represents the cross-sectional area of the edge of the film located in the path.

$$\%\ elongation\ at\ break = \frac{\sqrt{R^2 + d^2} - R}{R} \cdot 100\ \% \qquad\qquad (4)$$

Here, R denotes the radius of the film exposed in the cylindrical hole of the holder and d the displacement to puncture.

$$energy\ at\ break\ per\ unit\ volume = \frac{AUC}{V} \qquad\qquad (5)$$

where AUC is the area under the load versus displacement curve and V the volume of the film located in the die cavity of the film holder (the energy at break is normalized to the film's volume).

*Pellet coating*

Theophylline matrix cores were coated with different Aquacoat® ECD:guar gum blends. Aquacoat® ECD was plasticized for 1 day with 25 % DBS (w/w, based on the ethylcellulose content). Guar gum was dissolved in purified water (*very low* $\eta$: 2 %, *low* $\eta$: 1.5 %, *high* $\eta$: 1 %, *medium* $\eta$: 0.7 % or 1 %, as indicated, w/w; 100 % reference value = total coating formulation; 2 h stirring). The two liquids were blended and stirred for 30 min prior to use. If indicated, 10 or 50 % talc or 20 % glyceryl monostearate (GMS) was added to the coating formulation as anti-tacking agent (referred to the total polymer content). In the case of GMS, the latter was dispersed in a 0.08 % w/v aqueous solution of polysorbate 80 at 65 °C under stirring for 10 minutes prior to guar gum addition. The coating dispersions were sprayed onto theophylline pellets using a fluidized bed coater (Strea 1, Wurster insert; Niro; Aeromatic-Fielder, Bubendorf, Switzerland). The process parameters were as follows: inlet temperature = 38 °C, product temperature = 38 $\pm$ 2 °C, spray rate = 2g/min, atomization pressure = 1.2 bar, nozzle diameter = 1.2 mm. After coating the pellets were further fluidized for 10 minutes and subsequently cured for 24 hours at 60 °C in an oven.

*Drug release measurements*

Theophylline release from coated pellets was separately measured in each of 0.1 M HCl and 0.1 M HCl: ethanol 60:40, followed by phosphate buffer pH 7.4 (USP 32) using the USP 32 paddle apparatus (Sotax, Basel, Switzerland) (900 mL, complete medium change after 2 h; 37 °C, 100 rpm; n = 3). At pre-determined time points, 3 mL samples were withdrawn and analyzed UV-spectrophotometrically ($\lambda$ = 270.4 nm in 0.1 N HCl, $\lambda$ = 272.2 nm in 0.1 M HCl:ethanol 60:40 and phosphate buffer pH 7.4) (UV 1650 PC, Shimadzu, Champs-sur-Marne, France). Drug release was measured before (if not otherwise indicated) or after storage under ambient conditions (storage at 60 % relative humidity & 25 °C) or stress conditions (storage at 75 % relative humidity & 40 °C).

*Results and discussion*

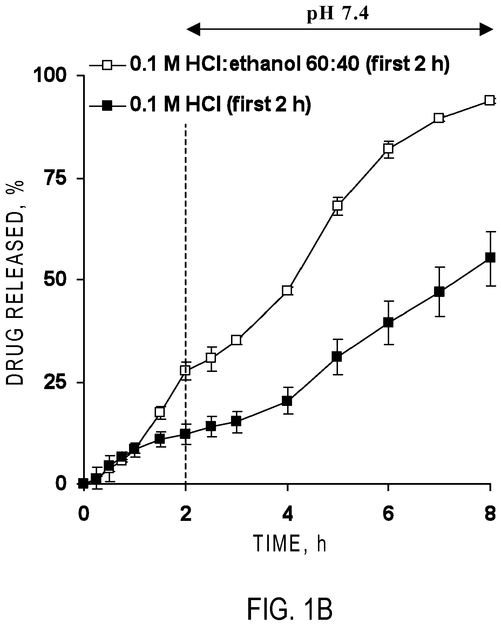

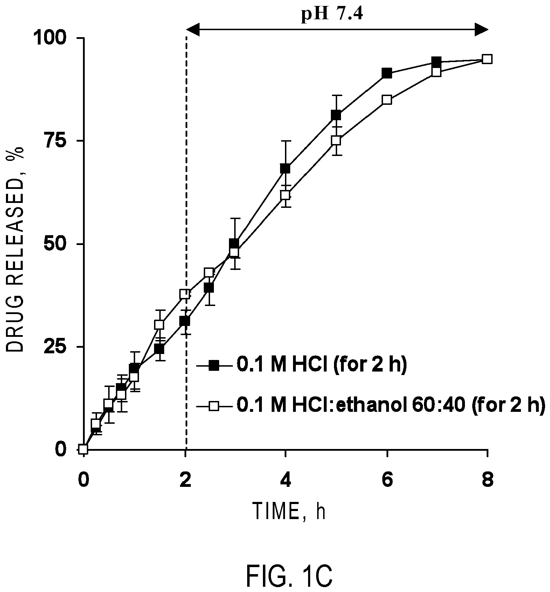

FIG. 1 shows the release of theophylline from drug matrix cores coated with ethylcellulose:guar gum 90:10 blends into: (i) 0.1 M HCl for 2 hours, followed by phosphate buffer pH 7.4 for 6 hours (filled squares), or (ii) 0.1 M HCl:ethanol 60:40 for 2 hours, followed by phosphate buffer pH 7.4 for 6 hours (open squares). The ethanol concentration was intentionally high to simulate "worst case" conditions *in vivo* (consumption of beverages with significant ethanol content). Three different types of guar gums were studied: (a) *very low* $\eta$ guar gum, (b) *low* $\eta$ guar gum, and (c) *high* $\eta$ guar gum (as defined hereinabove).

As it can be seen, the *very low* and *low* $\eta$ guar gum containing film coatings exhibited significant sensitivity to the presence of ethanol in the surrounding bulk fluid. Theophylline release was much faster in a 0.1 M HCl:ethanol 60:40 blend than in 0.1 M HCl (FIGS. 1A and 1B). As it can be seen in FIG. 1C, the difference between "drug release in 0.1 M HCl:ethanol 60:40" and "drug release in 0.1 M HCl" was negligible for film coatings based on ethylcellulose containing

10 % *high* $\eta$ guar gum.

Next, *medium* $\eta$ guar gum was tested with 10 % talc added to the coating formulation. FIG. 2 shows the resulting release kinetics of theophylline from pellets coated with 20 % ethylcellulose:*medium* $\eta$ guar gum 85:15, containing 10 % talc. The filled squares show drug release in 0.1 M HCl for 2 hours, followed by phosphate buffer pH 7.4 for the subsequent 6 hours. The open squares indicate theophylline release in 0.1 M HCl:ethanol 60:40 for 2 hours, followed by 6 hours in phosphate buffer pH 7.4. The resulting drug release rate was not significantly affected by the presence of 40 % ethanol in the release medium. Thus, the *medium* $\eta$ guar gum was found to be sufficient to allow for an effective hindering of ethylcellulose dissolution due to the presence of the guar gum network and sticking was reduced during coating.

To confirm the functionality of the invention, thin polymeric films consisting of ethylcellulose:*medium* $\eta$ guar gum 85:15 were prepared and their dry mass loss behavior monitored upon exposure to 0.1 M HCl and 0.1 M HCl:ethanol 60:40. As can be seen in FIG. 3, the dry mass loss of the systems was very similar and limited in both cases. Thus, the guar gum network of the present invention effectively hindered the dissolution of ethylcellulose, even in the presence of 40 % ethanol.

The compatibility of Aquacoat® ECD and guar gum was investigated and the appearance of thin films prepared from Aquacoat® ECD:guar gum blends studied. FIG. 4A shows as an example a microscopic picture of an Aquacoat® ECD:*medium* $\eta$ guar gum 85:15 blend, which was stirred for 24 hours at room temperature. As it can be seen, no signs of incompatibility (e.g., flocculation) were visible. Furthermore, macroscopic pictures of thin films prepared from Aquacoat® ECD:*medium* $\eta$ guar gum 85:15, which were cured at 60 °C for 24 hours, revealed homogeneous, crack-free systems with a smooth surface (FIG. 4B). Thus, the addition of small amounts of guar gum to Aquacoat® ECD does not impair the stability of the coating dispersion, nor the homogeneity of the resulting polymeric films.

Next, the mechanical properties of a controlled release film coating of the present invention were studied. Using a texture analyzer the puncture strength, % elongation at break and energy required to break thin, free films of identical composition as the film coatings were determined in the dry and wet state (see Table 1 below).

**Table 1**

Mechanical properties of thin polymeric films prepared from Aquacoat® ECD:*medium* $\eta$ guar gum (polymer:polymer blend ratio = 85:15) in the dry and wet state (in the latter case, films were exposed to a 60:40 mixture of 0.1 M HCl and ethanol for 0.5, 1 or 2 h, as indicated) (RT = room temperature).

|  | Dry (RT) | 0.5 h (37 °C) | 1 h (37 °C) | 2 h (37 °C) |
|---|---|---|---|---|
| Puncture strength, MPa | 0.62 ($\pm$ 0.06) | 0.32 ($\pm$ 0.03) | 0.27 ($\pm$ 0.06) | 0.19 ($\pm$ 0.08) |
| Elongation at break, % | 0.76 ($\pm$ 0.07) | 29.5 ($\pm$ 6.6) | 18.9 ($\pm$ 4.1) | 8.7 ($\pm$ 3.0) |
| Energy at break, MJ/m$^3$ | 0.02 ($\pm$ 0.00) | 0.09 ($\pm$ 0.01) | 0.06 ($\pm$ 0.02) | 0.03 ($\pm$ 0.01) |

As it can be seen, in all cases significant stability was provided. It has to be pointed out that the mechanical strength of a film coating should not only be determined in the dry state at room temperature (this is important to know about the risk of accidental crack formation during storage and transport), but also upon contact with the release media. On the one hand, compounds of the polymeric films (e.g., plasticizers) might leach out into the surrounding bulk fluids. On the other hand, water and/or ethanol might act as plasticizers for the polymeric compounds. As it can be seen in Table 1, the exposure of thin films prepared from Aquacoat® ECD:*medium* $\eta$ guar gum (polymer:polymer blend ratio = 85:15) led to a significant increase in the systems' flexibility (note that also the temperature was increased from room temperature to 37 °C when compared to the dry state). Thus, the presence of 40 % ethanol in the surrounding bulk fluid did not lead to crack formation and subsequent dose dumping.

In order to demonstrate the effectiveness of the anti-tack agent in the present invention (Aquacoat® ECD:*medium* $\eta$ guar gum was used in this test), the amount of the anti-tacking agent talc was increased to 50 % talc, and alternatively another type of anti-tacking agent was studied: glyceryl monostearate (GMS) at a contents of 20 % (referred to the total polymer content). Importantly, in both cases, the ethanol insensitivity of the controlled release film coatings was unaffected. FIG. 5 shows theophylline release from matrix pellets coated with Aquacoat® ECD:*medium* $\eta$ guar gum 90:10 containing these two anti-tacking agents in: (i) 0.1 M HCl (filled squares), or (ii) 0.1 M HCl:ethanol 60:40 (open squares) for 2 hours, followed (in both cases) by phosphate buffer pH 7.4 for 6 h. The resulting drug release profiles were virtually overlapping in the presence/absence of 40 % ethanol in the bulk fluid in both cases. The sticking was more effectively reduced when including 20 % GMS compared to 50 % talc.

FIG. 6 exemplarily shows theophylline release from matrix pellets coated with Aquacoat® ECD:*medium* $\eta$ guar gum 90:10 in 0.1 M HCl:ethanol 60:40 for 2 hours and phosphate buffer pH 7.4 for the subsequent 6 h (anti-tacking agent = talc, 50 %). The solid curve indicates drug release prior to storage and the dotted curves after 12 months open storage (without packaging material) under ambient and stress conditions. The release profiles are very similar. Thus, the presence of 10 % guar gum can effectively trap water within the polymeric system during coating and curing, facilitating polymer particle coalescence and/or sterically hinder further film formation during long term storage.

FIG. 7 shows the impact of the curing conditions on drug release from pellets loaded with theophylline and coated with 20 % w/w ethylcellulose *medium* $\eta$ guar gum 85:15 in 0.1 M HCl:ethanol 60:40 for 2 hours and phosphate buffer pH 7.4 for the subsequent 6 h. The curing conditions were as follows: 1 day at 60 °C & ambient relative humidity, or 2 days at 60 °C & ambient relative humidity, or 1 day at 60 °C and 75 % relative humidity, or 2 days at 60 °C & 75 % relative humidity (as indicated in FIG. 7). The resulting drug release profiles were similar, indicating that a stable film coating was achieved.

The impact of the curing conditions on the storage stability of ethylcellulose:*medium* $\eta$ guar gum 85:15 coated pellets (coating level: 20 % w/w) is illustrated by FIGS. 8A and 8B. Theophylline release is shown before storage (solid line curves; closed symbols) and after 6 months open storage (dotted line curves; open symbols) at: (a) ambient conditions (FIG. 8A), and (b) stress conditions (40 °C and 75% relative humidity) (FIG. 8B). The curing conditions were as follows: 1 day at 60 °C & ambient relative humidity, or 2 days at 60 °C & ambient relative humidity, or 1 day at 60 °C and 75 % relative humidity, or 2 days at 60 °C & 75 % relative humidity (as indicated). The release medium was 0.1 M HCl:ethanol 60:40 for the first 2 h, followed by phosphate buffer pH 7.4 for the subsequent 6 h. As it can be seen, in all cases drug release before storage was similar to drug release after storage, irrespective of the curing conditions and storage conditions. This clearly indicates that stable film coatings were achieved.

The reproducibility of the film coating process is illustrated in FIG. 9. Theophylline release from three different pellet batches (which were coated with 20 % w/w ethylcellulose:*medium* $\eta$ guar gum 85:15) is shown in 0.1 M HCl for 2 h, followed by phosphate buffer pH 7.4 (closed symbols) or in 0.1 M HCl:ethanol 60:40 for 2 h, followed by phosphate buffer pH 7.4 (open symbols). The resulting drug release kinetics were similar, indicating that the film coating process had good reproducibility.

The robustness of drug release from theophylline loaded pellets coated with 20 % w/w ethylcellulose:*medium* $\eta$ guar gum 85:15 with respect to the degree of agitation of the bulk fluid is shown in FIG. 10. Drug release is shown in 0.1 M HCl for 2 h, followed by phosphate buffer pH 7.4 (closed symbols) or in 0.1 M HCl:ethanol 60:40 for 2 h, followed by phosphate buffer pH 7.4 (open symbols) at different agitation speeds: 50, 75 or 100 rpm (as indicated). As it can be seen, the resulting drug release rates were similar, indicating that drug release from these systems was robust with respect to variations in mechanical stress. This is desirable with respect to the reproducibility of drug release within the gastro intestinal tract (wherein the pellets can be exposed to different degrees of mechanical stress due to variations in the motility and contents of the gastro intestinal tract).

The impact of the exposure time to elevated ethanol concentrations in the release medium on drug release is illustrated in FIG. 11. Theophylline release is shown from pellets coated with 20 % w/w ethylcellulose:medium $\eta$ guar gum 85:15 in 0.1 M HCl:ethanol 60:40 for 2 h, followed by phosphate buffer pH 7.4 (closed symbols) or in 0.1 M HCl:ethanol 60:40 for 8 h (open symbols). The resulting drug release kinetics were very similar, indicating that the exposure time to elevated ethanol concentrations did not meaningfully affect the performance of the controlled drug delivery systems in the gastro intestinal tract. This is important given the possible variation in exposure times.

FIGS. 12A-12F show the impact of the ethylcellulose:*medium* $\eta$ guar gum blend ratio (i.e., at 70:30, 85:15, 90:10, 93:7, 95:5, and 97:3) on the sensitivity of drug release to ethanol. Theophylline release was measured from pellets (15 % coating level) in 0.1 M HCl for 2 h, followed by phosphate buffer pH 7.4 (closed symbols) or in 0.1 M HCl:ethanol 60:40 for 2 h, followed by phosphate buffer pH 7.4 (open symbols). As is seen in FIGS. 12A-12F, the blends in amounts within the scope of the invention (FIGS. 12A-12D) were not sensitive to the presence of ethanol in the release medium while the blends in amounts outside the invention (FIGS. 12E-12F) were sensitive to ethanol.

The morphology of the surface of pellets coated with different ethylcellulose:*medium* $\eta$ guar gum blend ratios (93:7, 90:10 or 85:15, as indicated) is shown in FIG. 13. Scanning electron microscopy pictures of pellets before exposure to the release medium are shown. As can be seen, the film coatings did not show any indication of cracks or inhomogeneous coatings.

FIGS. 14A-14C show macroscopic pictures of free, polymeric films consisting of ethylcellulose:*medium* $\eta$ guar gum 85:15 at t=0 and after exposure to 0.1 M HCl or 0.1 M HCl:ethanol 60:40 for 2 h (and subsequent drying), as indicated. The films were homogeneous and after exposure to the release media no indication for macropore formation or any inhomogeneity was visible, irrespective of the presence or absence of ethanol in the release medium.

The liquid (water plus ethanol) uptake and dry mass loss of thin, free ethylcellulose:*medium* $\eta$ guar gum films based on 85:15, 90:10, or 93:7 blends upon exposure to 0.1 M HCl:ethanol 60:40 is shown in FIGS. 15A and 15B. As can be seen, the liquid uptake was similar for all blend ratios and the dry mass loss was very limited in all cases. This is a further confirmation that such film coatings were able to provide controlled drug release, also in the presence of significant amounts of ethanol.

FIG. 16 illustrates the impact of the ethanol content in the bulk fluid (0, 5, 10, 20 or 40 %, as indicated in the diagram) on theophylline release from pellets coated with 20 % w/w ethylcellulose:*medium* $\eta$ guar gum 93:7 (1 % guar gum in total dispersion). The release medium was a 0.1 M HCl:ethanol mixture for the first 2 h, followed by phosphate buffer pH 7.4 for the subsequent 6 h. Importantly, there is no significant effect of the ethanol content of the release medium on the resulting drug release kinetics. Thus, variations in the ethanol content in the gastro intestinal tract in the patient can be expected to have a negligible effect on the performance of this type of advanced drug delivery system *in vivo.* This is of great practical importance, since the ethanol content in the gastro intestinal tract can significantly vary.

FIGS. 17A and 17B show the impact of the coating level (10, 15, or 20 %, as indicated in the figures) on theophylline release from pellets coated with:

a) ethylcellulose:*medium* $\eta$ guar gum 85:15 (0.7 % guar gum in total dispersion; FIG. 17A), or b) 93:7 (1 % guar gum in total dispersion; FIG. 17B) in 0.1 M HCl for 2 h, followed by phosphate buffer pH 7.4 (closed symbols) or 0.1 M HCl:ethanol 60:40 for 2 h, followed by phosphate buffer pH 7.4 (open symbols). As it can be seen, drug release at the tested coating levels was not sensitive to the presence of ethanol in the release medium and, if desired, the resulting drug release rate can be further modified by varying the coating level. This is of great practical importance.

The storage stability of theophylline pellets coated with 20% w/w (as indicated) ethylcellulose:*medium* $\eta$ guar gum 85:15 is illustrated in FIGS. 18A and 18B. Drug release is shown before (solid curves, closed symbols) and after 6 and 12 months open storage (dotted curves, open symbols) under: (a) ambient conditions (25 °C and 40 % relative humidity), or (b) stress conditions (40 °C and 75 % relative humidity) in 0.1 M HCl:ethanol 60:40 for 2 h, followed by phosphate buffer pH 7.4. Importantly, in all cases the drug release was similar before and after storage. Thus, the inventive film coatings were stable, irrespective of the storage conditions and coating level. Again, this is of great practical importance.

FIGS. 19A and 19B show the effects of the dilution of the coating dispersion on: (a) the viscosity of the formulation sprayed onto the pellets (FIG. 19A), and (b) the percentage of *medium* $\eta$ guar gum with respect to the "guar gum + total water" content of the formulation (FIG. 19B). The bars indicate the ethylcellulose:*medium* $\eta$ guar gum blend ratio (93:7, 90:10 and 85:15 w/w, respectively). Importantly, all measured viscosities allowed for convenient processing. The viscosity of the coating formulation increased with increasing guar gum content (FIG. 19A). For a given guar gum percentage (100 % = total dispersion), the viscosity of the formulation decreased when changing the ethylcellulose:*medium* $\eta$ guar gum blend ratio from 93:7 to 90:10 and to 85:15. This can be explained by the decreasing percentage of guar gum with respect to the "guar gum + water" reference value (FIG. 19B).

FIGS. 20A-20D show the impact of the guar gum dilution (0.7% versus 1%) on theophylline release from pellets coated with different ethylcellulose:*medium* $\eta$ guar gum blends (as indicated; coating level: 20 % w/w) in 0.1 M HCl for 2 h, followed by phosphate buffer pH 7.4 (closed symbols) or 0.1 M HCl:ethanol 60:40 for 2 h, followed by phosphate buffer pH 7.4 (open symbols). Importantly in all tested cases the resulting drug release kinetics of the invention was not sensitive to the presence of ethanol in the release medium, irrespective of the tested ethylcellulose:guar gum blend ratio and percentage of guar gum in the coating formulation.

FIGS. 21A-21B show theophylline release from *single* pellets (n = 6) coated with 20 % w/w ethylcellulose:*medium* $\eta$ guar gum 85:15 in: a) 0.1 M HCl for 2 h, followed by phosphate buffer pH 7.4, and b) 0.1 M HCl:ethanol 60:40 for 2 h, followed by phosphate buffer pH 7.4. The thick curves show the respective mean values, error bars indicate standard deviations. As can be seen, there were no significant differences in the drug release profiles between the tested single pellets; thus, the underlying drug release mechanism was uniform.

FIG. 22 shows the impact of the osmolality of the release medium (indicated in the diagram) the pellets are exposed to on drug release from the systems. Theophylline release is shown from pellets coated with 20 % w/w ethyl-cellulose:*medium* $\eta$ guar gum 85:15 in 0.1 M HCl containing different amounts of NaCl for 2 h, followed by phosphate buffer pH 7.4. Importantly, there was no major impact of the osmolality of the release medium on drug release. Thus, the formation of cracks (due to the creation of significant hydrostatic pressure within the cores) was not seen.

The absence of cracks within the film coatings after exposure to the release medium has further been confirmed by scanning electron microscopy (SEM). FIGS. 23A-23C show SEM pictures of surfaces of pellets, which were coated with 20 % w/w ethylcellulose:*medium* $\eta$ guar gum 85:15 before and after exposure to 0.1 M HCl or 0.1 M HCl:ethanol 60:40 for 2 h (and subsequent drying) (as indicated). Importantly, no cracks were visible, irrespective of the presence or absence of ethanol in the release medium.

Since the potential creation of cracks within the film coatings during drug release can be strongly dependent on the mechanical stability of the film coatings, the mechanical properties of the latter were measured. FIGS. 24A-24F show the mechanical properties of free, ethylcellulose:*medium* $\eta$ guar gum films in the dry state at room temperature (FIGS. 24A, 24C and 24E) and in the wet state at 37 °C (FIGS. 24B, 24D and 24F) after exposure to 0.1 M HCl:ethanol 60:40 for different time periods (as indicated). The ethylcellulose:*medium* $\eta$ guar gum blend ratio was varied from 93:7 to 90:10 to 85:15, as indicated in the diagrams. Importantly, in all cases, the values obtained indicate that these film coatings withstand the mechanical stress the pellets may be exposed to in the gastro intestinal tract, irrespective of the investigated ethylcellulose:guar gum blend ratio and exposure time to the release medium. Thus, crack formation during drug release with the invention was not seen.

*Conclusion*

The results of the foregoing tests indicate that solid dosage forms coated with the coating formulation of the present invention showed ethanol resistance, while the solid dosage forms coated with the comparative film coating compositions (e.g., containing ethylcellulose and guar gums having lower apparent viscosities than those of the present invention), as well as those coating blends using the guar gum of the invention but in amounts outside the invention, showed significant ethanol sensitivity. This is of great practical importance, for example, for highly potent drugs (where dose dumping is particularly unacceptable).

## EXAMPLE 2

Theophylline matrix pellets were coated with a blend of 90 % ethylcellulose and 10 % guar gum, the latter being a blend of *medium $\eta$* and *low $\eta$* guar gum (as defined above). The coatings were prepared and the pellets were coated as described above. The coating level was 20 %. The *medium $\eta$: low $\eta$* guar gum blend ratio was varied as indicated below in order to compare the functionality of several different guar gum blends. The apparent viscosities were measured in the same way as the viscosities of the single guar gums in Example 1 (i.e., at a shear rate of 50 s$^{-1}$ in a 1% aqueous guar gum solution measured rotationally at 20°C after 1 minute equilibration using a 6 cm acrylic cone (1°) on a cone-plate viscometer wherein the shear is ramped up linearly from 1 to 50 s$^{-1}$ in 25 steps over 29 seconds). The results are set forth immediately below.

| Blend ratio (weight: weight) *medium $\eta$: low $\eta$* guar gum | Viscosity $\pm$ SD of the guar gum blend (n = 3) (cps) | Drug release in 0.1 N HCl after 2 h (%) | Difference in drug release after 2 h exposure to "0.1 N HCl" versus "0.1 M HCl:ethanol 60:40" (%) | Alcohol sensitivity |
|---|---|---|---|---|
| 90:10 | 303.5 $\pm$ 2.1 | 52.9 | 5.9 | No |
| 80:20 | 276.0 $\pm$ 1.4 | 63.6 | 5.3 | No |
| 75:25 | 260.0 $\pm$ 7.2 | 49.8 | 8.8 | No |
| 70:30 | 234.0 $\pm$ 7.6 | 48.1 | 4.9 | No |
| 50:50 | 173.6 $\pm$ 3.1 | 40.1 | 14.3 | No |
| 47.5:52.5 | 160.0 $\pm$ 6.2 | 51.4 | 15.1 | No |
| 45:55 | 151.0 $\pm$ 2.7 | 45.6 | 25.1 | No |
| 40:60 | 148.5 $\pm$ 2.1 | 37.9 | 42.1 | Yes |

As can be seen from the foregoing table, the sample having a guar gum apparent viscosity of 148.5$\pm$ 2.1 cps (outside the scope of the invention) was alcohol sensitive whereas all the other samples tested were within the scope of the present invention and found to be alcohol insensitive.

## Claims

1. A solid dose form comprising a film coating composition encapsulating a core, wherein:

   (i) said core comprises an active ingredient comprising at least one of a pharmaceutical, veterinary, or nutraceutical active ingredient;
   (ii) said film coating composition comprises ethylcellulose and guar gum,
   (iii) said guar gum is present in an amount greater than 5 wt% based on the weight of the guar gum and ethylcellulose; **characterized in that**
   (iv) said guar gum has an apparent viscosity $\geq$ 151.0 cps at a shear rate of 50 s$^{-1}$ in a 1% aqueous guar gum solution measured rotationally at 20°C after 1 minute equilibration using a 6 cm acrylic cone (1°) on a cone-plate viscometer wherein the shear is ramped up linearly from 1 to 50 s$^{-1}$ in 25 steps over 29 seconds;
   (v) said dose form provides sustained release of said active ingredient; and
   (vi) said dose form is ethanol resistant **in that** the release kinetics of said active ingredient are not significantly affected by the presence of alcohol, as determined by measuring a dissolution profile of said active ingredient in a USP 32 paddle apparatus operated with 900 ml at 37 °C and 100 rpm.

**2.** The solid dose form of claim 1, wherein the difference between the release profile of said active ingredient in (i) 0.1M HCl for two hours at 37 °C and subsequently in phosphate buffer at pH 7.4 for three hours at 37 °C and (ii) 0.1M HCl and 40% ethanol at 37 °C for two hours and subsequently in phosphate buffer at pH 7.4 for at least three hours at 37 °C is less than 15% when less than 20% of the active is released in ethanol free media and less than 30% when greater than 20 to 40% of the active is released in the ethanol free media.

**3.** The solid dose form of claim 1, wherein a wt % ratio of said ethylcellulose to guar gum is from 60:40 to less than 95:5, respectively.

**4.** The solid dose form of claim 3, wherein said ratio is 80:20 to 92:8, respectively.

**5.** The solid dose form of claim 3, wherein said ratio is 85:15 to 90:10, respectively.

**6.** The solid dose form of claim 3, wherein said ratio is 90:10, respectively.

**7.** The solid dose form of claim 1, wherein said film coating composition further comprises at least one of a surfactant or anti-tack agent.

**8.** The solid dose form of claim 1, wherein said film coating composition is present on a dry solids basis in an amount of from 2 to 40 % by weight of the total dose form.

**9.** The solid dose form of claim 1, wherein said core is a pellet, tablet, capsules, granule, films, any solid coated dosage form.

**10.** A film coating composition comprising ethylcellulose and guar gum, **characterized in that**:

(i) said guar gum has an apparent viscosity $\geq$ 151.0 cps at a shear rate of 50 s$^{-1}$ in a 1% aqueous guar gum solution measured rotationally at 20°C after 1 minute equilibration using a 6 cm acrylic cone (1°) on a cone-plate viscometer, wherein the shear is ramped up linearly from 1 to 50 s$^{-1}$ in 25 steps over 29 seconds;
(ii) said film coating composition provides sustained release of a pharmaceutical, veterinary or nutraceutical active ingredient contained within a solid dose form containing said film coating composition; and
(iii) said film coating composition provides ethanol resistance to a pharmaceutical, veterinary or nutraceutical active ingredient contained within a solid dose form containing said film coating composition, **in that** the release kinetics of said active ingredient are not significantly affected by the presence of alcohol, as determined by measuring a dissolution profile of said active ingredient in a USP 32 paddle apparatus operated with 900 ml at 37 °C and 100 rpm.

**11.** The solid dose form of claim 1 or the film coating composition of claim 10, wherein said viscosity of said guar gum is from 151.0 cps to 2,000 cps.

**12.** The solid dose form of claim 1 or the film coating composition of claim 10, wherein a wt % ratio of said ethylcellulose to guar gum is from 70:30 to 93:7, respectively.

**13.** The solid dose form of claim 1 or the film coating composition of claim 10, wherein the film coating composition further comprising a plasticizer.

**14.** The solid dose form of claim 1 or the film coating composition of claim 10, wherein said guar gum has an apparent viscosity greater than 250 cps.

**15.** A method of reducing the ethanol sensitivity of a pharmaceutical, nutraceutical or veterinary active ingredient in the core of a solid dosage form comprising coating said core with a film coating composition comprising the film coating composition of claim 10, wherein said film coating composition provides said sustained release of and said ethanol resistance to said active ingredient.

**Patentansprüche**

**1.** Eine feste Dosisform umfassend eine Filmbeschichtungszusammensetzung, welche einen Kern umkapselt, wobei:

(i) besagter Kern einen Wirkstoff umfasst, der mindestens einen pharmazeutischen, veterinären oder nutrazeutischen Wirkstoff umfasst;

(ii) besagte Filmbeschichtungszusammensetzung Ethylcellulose und Guargummi umfasst;

(iii) besagtes Guargummi in einer Menge vorhanden ist, die größer ist als 5 Gew.-% bezogen auf das Gewicht des Guargummis und der Ethylcellulose; **dadurch gekennzeichnet, dass**

(iv) besagtes Guargummi eine scheinbare Viskosität von $\geq$ 151,0 cps aufweist bei einer Schergeschwindigkeit von 50 s$^{-1}$ in einer 1 %igen wässrigen Guargummilösung rotational gemessen bei 20 °C nach 1-minütiger Äquilibrierung unter Verwendung eines 6-cm-Acrylkegels (1°) auf einem Kegel-Platte-Viskosimeter, wobei die Schergeschwindigkeit innerhalb von 29 Sekunden in 25 Schritten linear von 1 auf 50 s$^{-1}$ erhöht wird;

(v) besagte Dosisform eine anhaltende Freisetzung des besagten Wirkstoffes bereitstellt; und

(vi) besagte Dosisform ethanolresistent ist, insofern als die Freisetzungskinetik des besagten Wirkstoffes nicht signifikant durch die Präsenz von Alkohol beeinflusst wird, gemäß Messung eines Auflösungsprofils des besagten Wirkstoffes in einer USP-32-Paddle-Apparatur betrieben mit 900 ml bei 37 °C und 100 UPM.

2. Die feste Dosisform gemäß Anspruch 1, wobei die Differenz zwischen dem Freisetzungsprofil des besagten Wirkstoffes in (i) 0,1 M HCl für zwei Stunden bei 37 °C und nachfolgend in Phosphatpuffer bei pH 7,4 für drei Stunden bei 37 °C und (ii) 0,1 M HCl und 40 % Ethanol bei 37 °C für zwei Stunden und nachfolgend in Phosphatpuffer bei pH 7,4 für mindestens drei Stunden bei 37 °C weniger als 15 % beträgt, wenn weniger als 20 % des Wirkstoffes in ethanolfreien Medien freigesetzt wird, und weniger als 30 %, wenn mehr als 20 bis 40 % des Wirkstoffes in den ethanolfreien Medien freigesetzt wird.

3. Die feste Dosisform gemäß Anspruch 1, wobei ein Gew.-%-Verhältnis der besagten Ethylcellulose zum Guargummi von 60:40 bis respektive weniger als 95:5 beträgt.

4. Die feste Dosisform gemäß Anspruch 3, wobei das besagte Verhältnis 80:20 bis respektive 92:8 beträgt.

5. Die feste Dosisform gemäß Anspruch 3, wobei das besagte Verhältnis 85:15 bis respektive 90:10 beträgt.

6. Die feste Dosisform gemäß Anspruch 3, wobei das besagte Verhältnis respektive 90:10 beträgt.

7. Die feste Dosisform gemäß Anspruch 1, wobei die besagte Filmbeschichtungszusammensetzung ferner mindestens ein Tensid oder ein Antihaftmittel umfasst.

8. Die feste Dosisform gemäß Anspruch 1, wobei die besagte Filmbeschichtungszusammensetzung auf einer Trockenfeststoff-Basis in einer Menge von 2 bis 40 Gew.-% der Gesamtdosisform vorhanden ist.

9. Die feste Dosisform gemäß Anspruch 1, wobei der besagte Kern ein Pellet, eine Tablette, Kapseln, Granulat, Filme, eine beliebige feste beschichtete Dosisform ist.

10. Eine Filmbeschichtungszusammensetzung umfassend Ethylcellulose und Guargummi, **dadurch gekennzeichnet, dass**:

(i) besagtes Guargummi eine scheinbare Viskosität von $\geq$ 151,0 cps aufweist bei einer Schergeschwindigkeit von 50 s$^{-1}$ in einer 1%igen wässrigen Guargummilösung rotational gemessen bei 20 °C nach 1-minütiger Äquilibrierung unter Verwendung eines 6-cm-Acrylkegels (1°) auf einem Kegel-Platte-Viskosimeter, wobei die Schergeschwindigkeit innerhalb von 29 Sekunden in 25 Schritten linear von 1 auf 50 s$^{-1}$ erhöht wird;

(ii) besagte Filmbeschichtungszusammensetzung eine anhaltende Freisetzung eines pharmazeutischen, veterinären oder neutrazeutischen Wirkstoffes bereitstellt, welcher in einer festen Dosisform enthalten ist, welche die besagte Filmbeschichtungszusammensetzung enthält; und

(iii) besagte Filmbeschichtungszusammensetzung Ethanolresistenz für einen pharmazeutischen, veterinären oder neutrazeutischen Wirkstoff bereitstellt, welcher in einer festen Dosisform enthalten ist, welche die besagte Filmbeschichtungszusammensetzung enthält, insofern als die Freisetzungskinetik des besagten Wirkstoffes nicht signifikant durch die Präsenz von Alkohol beeinflusst wird, gemäß Messung eines Auflösungsprofils des besagten Wirkstoffes in einer USP-32-Paddle-Apparatur betrieben mit 900 ml bei 37 °C und 100 UPM.

11. Die feste Dosisform gemäß Anspruch 1 oder die Filmbeschichtungszusammensetzung gemäß Anspruch 10, wobei besagte Viskosität des besagten Guargummis von 151,0 cps bis 2.000 cps beträgt.

**12.** Die feste Dosisform gemäß Anspruch 1 oder die Filmbeschichtungszusammensetzung gemäß Anspruch 10, wobei ein Gew.-%-Verhältnis der besagten Ethylcellulose zum besagten Guargummi von 70:30 bis respektive 93:7 beträgt.

**13.** Die feste Dosisform gemäß Anspruch 1 oder die Filmbeschichtungszusammensetzung gemäß Anspruch 10, wobei die Filmbeschichtungszusammensetzung ferner einen Weichmacher umfasst.

**14.** Die feste Dosisform gemäß Anspruch 1 oder die Filmbeschichtungszusammensetzung gemäß Anspruch 10, wobei besagtes Guargummi eine scheinbare Viskosität von mehr als 250 cps aufweist.

**15.** Ein Verfahren zur Reduktion der Ethanolempfindlichkeit eines pharmazeutischen, veterinären oder neutrazeutischen Wirkstoffes im Kern einer festen Dosierungsform umfassend die Beschichtung des besagten Kerns mit einer Filmbeschichtungszusammensetzung, welche die Filmbeschichtungszusammensetzung gemäß Anspruch 10 umfasst, wobei besagte Filmbeschichtungszusammensetzung die besagte anhaltende Freisetzung des besagten Wirkstoffes sowie die besagte Ethanolresistenz des besagten Wirkstoffes bereitstellt.

## Revendications

**1.** Une forme galénique solide comprenant une composition d'enrobage pelliculaire encapsulant un noyau, **caractérisée en ce que** :

(i) ledit noyau comprend un ingrédient actif comprenant au moins un ingrédient actif pharmaceutique, vétérinaire ou nutraceutique ;
(ii) ladite composition d'enrobage pelliculaire comporte de l'éthylcellulose et de la gomme de guar ;
(iii) ladite gomme de guar est présente en quantité supérieure à 5 % en poids sur la base du poids de la gomme de guar et de l'éthylcellulose ; **caractérisée en ce que**
(iv) ladite gomme de guar présente une viscosité apparente supérieure ou égale à 151,0 cP à un taux de cisaillement de 50 s$^{-1}$ dans une solution aqueuse à 1 % de gomme de guar, mesurée en rotation à 20 °C après une minute de mise à l'équilibre sur un viscosimètre cône-plan équipé d'un cône en acrylique de 6 cm (1°), le cisaillement augmentant de façon linéaire de 1 à 50 s$^{-1}$ en 25 étapes en l'espace de 29 secondes ;
(v) ladite forme galénique assure la libération prolongée dudit ingrédient actif ; et
(vi) ladite forme galénique est résistante à l'éthanol **en ce que** la cinétique de libération dudit ingrédient actif n'est pas affectée de manière significative par la présence d'alcool, telle que déterminée en mesurant un profil de dissolution dudit ingrédient actif dans un agitateur à ailettes USP 32 en utilisant 900 ml à 37 °C et 100 tr/min.

**2.** La forme galénique solide selon la revendication 1, **caractérisée en ce que** la différence entre le profil de libération dudit ingrédient actif dans i) HCl à 0,1 M pendant deux heures à 37 °C puis dans un tampon de phosphate à pH 7,4 pendant trois heures à 37 °C et ii) HCl à 0,1 M et 40 % d'éthanol à 37 °C pendant deux heures puis dans un tampon de phosphate à pH 7,4 pendant au moins trois heures à 37 °C est inférieure à 15 % quand moins de 20 % de l'ingrédient actif est libéré dans le milieu sans éthanol, et inférieure à 30 % lorsque plus de 20 à 40 % de l'ingrédient actif est libéré dans le milieu sans éthanol.

**3.** La forme galénique solide selon la revendication 1, **caractérisée en ce que** le rapport de poids éthylcellulose-gomme de guar exprimé en pourcentage est supérieur à 60:40 et inférieur à 95:5, respectivement.

**4.** La forme galénique solide selon la revendication 3, **caractérisée en ce que** ledit rapport de poids est compris entre 80:20 et 92:8, respectivement.

**5.** La forme galénique solide selon la revendication 3, **caractérisée en ce que** ledit rapport de poids est compris entre 85:15 et 90:10, respectivement.

**6.** La forme galénique solide selon la revendication 3, **caractérisée en ce que** ledit rapport de poids est égal à 90:10.

**7.** La forme galénique solide selon la revendication 1, **caractérisée en ce que** ladite composition d'enrobage pelliculaire comprend en outre au moins un agent tensioactif ou antiadhérent.

**8.** La forme galénique solide selon la revendication 1, **caractérisée en ce que** ladite composition d'enrobage pelliculaire représente 2 à 40 % en poids de matière sèche de la forme galénique solide totale.

**9.** La forme galénique solide selon la revendication 1, **caractérisée en ce que** ledit noyau est une forme galénique solide enrobée, une pastille, un comprimé, une gélule, un granulé, un film.

**10.** Une composition d'enrobage pelliculaire comprenant de l'éthylcellulose et de la gomme de guar, **caractérisée en ce que** :

(i) ladite gomme de guar présente une viscosité apparente supérieure ou égale à 151,0 cP à un taux de cisaillement de 50 s$^{-1}$ dans une solution aqueuse à 1 % de gomme de guar, mesurée en rotation à 20 °C après une minute de mise à l'équilibre sur un viscosimètre cône-plan équipé d'un cône en acrylique de 6 cm (1°), le cisaillement augmentant de façon linéaire de 1 à 50 s$^{-1}$ en 25 étapes en l'espace de 29 secondes ;

(ii) ladite composition d'enrobage pelliculaire assure la libération prolongée d'un ingrédient actif pharmaceutique, vétérinaire ou nutraceutique contenu dans une forme galénique solide contenant ladite composition d'enrobage pelliculaire ; et

(iii) ladite forme galénique assure la résistance à l'éthanol d'un ingrédient actif pharmaceutique, vétérinaire ou nutraceutique contenu dans une forme galénique solide contenant ladite composition d'enrobage pelliculaire, **en ce que** la cinétique de libération dudit ingrédient actif n'est pas affectée de manière significative par la présence d'alcool, tel que déterminée en mesurant un profil de dissolution dudit ingrédient actif dans un agitateur à ailettes USP 32 en utilisant 900 ml à 37 °C et 100 tr/min.

**11.** La forme galénique solide selon la revendication 1 ou la composition d'enrobage pelliculaire selon la revendication 10, **caractérisée en ce que** ladite viscosité de ladite gomme de guar est comprise entre 151,1 et 2000 cP.

**12.** La forme galénique solide selon la revendication 1 ou la composition d'enrobage pelliculaire selon la revendication 10, **caractérisée en ce que** le rapport de poids éthylcellulose-gomme de guar exprimé en pourcentage est compris entre 70:30 et 93:7, respectivement.

**13.** La forme galénique solide selon la revendication 1 ou la composition d'enrobage pelliculaire selon la revendication 10, **caractérisée en ce que** la composition d'enrobage pelliculaire comprend en outre un plastifiant.

**14.** La forme galénique solide selon la revendication 1 ou la composition d'enrobage pelliculaire selon la revendication 10, **caractérisée en ce que** ladite gomme de guar présente une viscosité apparente supérieure à 250 cP.

**15.** Une méthode de réduction de la sensibilité à l'éthanol d'un ingrédient actif pharmaceutique, vétérinaire ou nutraceutique contenu dans le noyau d'une forme galénique solide comprenant l'enrobage dudit noyau dans une composition d'enrobage pelliculaire comprenant la composition d'enrobage pelliculaire selon la revendication 10, **caractérisée en ce que** ladite composition d'enrobage pelliculaire assure la libération prolongée et la résistance à l'éthanol dudit ingrédient actif.

FIG. 1A

FIG. 1B

FIG. 1C

FIG. 2

FIG. 3

10 μm

FIG. 4A

1 mm

FIG. 4B

FIG. 5A

FIG. 5B

FIG. 6

FIG. 7

FIG. 8A

FIG. 8B

EP 2 714 015 B1

FIG. 9

FIG. 10

FIG. 11

FIG. 12A

FIG. 12B

EP 2 714 015 B1

FIG. 12C

FIG. 12D

EP 2 714 015 B1

FIG. 12E

FIG. 12F

**93:7**

FIG. 13A

**90:10**

FIG. 13B

**85:15**

FIG. 13C

t = 0

1 mm

FIG. 14A

2 h 0.1 M HCl

200 µm

FIG. 14B

2 h 0.1 M HCl:
ethanol 60:40

200 µm

FIG. 14C

FIG. 15A

FIG. 15B

EP 2 714 015 B1

FIG. 16

FIG. 17A

FIG. 17B

EP 2 714 015 B1

40 °C/75 % RH

DRUG RELEASED, %

TIME, h

- before storage
- after 6 months storage
- after 12 months storage

FIG. 18B

25 °C/40 % RH

DRUG RELEASED, %

TIME, h

- before storage
- after 6 months storage
- after 12 months storage

FIG. 18A

FIG. 19A

FIG. 19B

EP 2 714 015 B1

FIG. 20A

FIG. 20B

0.7% GUAR GUM
IN TOTAL DISPERSION
85:15

FIG. 20C

1% GUAR GUM
IN TOTAL DISPERSION
85:15

FIG. 20D

EP 2 714 015 B1

**0.1 M HCl**

FIG. 21A

**0.1 M HCl:ethanol 60:40**

FIG. 21B

EP 2 714 015 B1

FIG. 22

t = 0

FIG. 23A

2 h 0.1 M HCl

FIG. 23B

2 h 0.1 M HCl:
ethanol 60:40

FIG. 23C

DRY STATE, RT

WET STATE, 37 °C

FIG. 24A

FIG. 24B

EP 2 714 015 B1

DRY STATE, RT

WET STATE, 37 °C

FIG. 24C

FIG. 24D

EP 2 714 015 B1

DRY STATE, RT

FIG. 24E

WET STATE, 37 °C

FIG. 24F

EP 2 714 015 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2007212414 A1

- US 7829148 B

**Non-patent literature cited in the description**

- Database. NLM17702405
- **JI CHONG-MIN et al.** Guar gum/ethylcellulose coated pellets for colon-specific drug delivery. *Yao Xue Xue Bao = ACTA Pharmaceutica Sinica,* June 2007, vol. 42 (6), 656-662
- **CAVALCANTI.** Characterisation of ethylcellulose films containing natural polysaccharides by thermal analysis and FTIR spectroscopy. *Acta Farm Bonaerense,* 01 January 2004

- **MEYER et al.** Awareness Topic: Mitigating the Risks of Ethanol Induced Dose Dumping From Oral Sustained/Controlled Release Dosage Forms. *FDA's ACPS Meeting,* October 2005
- **ROTH et al.** Ethanol Effects on Drug Release From Verapamil Meltrex, an Innovative Melt Extruded Formulation. *Int. J. Pharm.,* 2009, vol. 368, 72-75